(19) Europäisches Patentamt
European Patent Office
Office européen des brevets

(11) Publication number: **0 169 033 B1**

(12) # EUROPEAN PATENT SPECIFICATION

(45) Date of publication of patent specification: **12.02.92**

(51) Int. Cl.⁵: **C07C 59/60**, C07C 327/18, C07C 327/36, C07C 69/734, C07C 43/178, C07C 229/30, C07D 309/00

(21) Application number: **85304967.4**

(22) Date of filing: **11.07.85**

(54) **Inhibitors of slow reacting substance of anaphylaxis.**

(30) Priority: **18.07.84 US 632143**

(43) Date of publication of application:
**22.01.86 Bulletin 86/04**

(45) Publication of the grant of the patent:
**12.02.92 Bulletin 92/07**

(84) Designated Contracting States:
**AT BE CH DE FR GB IT LI NL SE**

(56) References cited:
**EP-A- 0 153 009**
**US-A- 2 602 816**
**US-A- 3 217 004**

**CHEMICAL ABSTRACTS, vol. 89, no. 16, 16th october 1978, page 36, abstract no. 130417q, Columbus, Ohio, US; & JP-A-78 30 653 (SANKYO ORGANIC CHEMICALS CO., LTD) 23-03-1978**

(73) Proprietor: **SCHERING CORPORATION**
**2000 Galloping Hill Road**
**Kenilworth New Jersey 07033(US)**

(72) Inventor: **Saksena, Anil Kumar**
**53 Beverly Road**
**Upper Montclair, NJ 07043(US)**
Inventor: **Wong, Jesse Kwok-Keung**
**547 Andress Terrace**
**Union, NJ 07083(US)**
Inventor: **Mangiaracina, Pietro**
**4 Montclair Avenue**
**Monsey, NY 10952(US)**

(74) Representative: **Ritter, Stephen David et al**
**Mathys & Squire 10 Fleet Street**
**London EC4Y 1AY(GB)**



Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

CHEMICAL ABSTRACTS, vol, 68, no. 11, 11th March 1968, pages 4722,4723, abstract no. 48956x, Columbus, Ohio, US; W. STOFFEL et al.: "Metabolism of sphingosine bases. III. Chemical syntheses of 14C- and 3H-labeled erythro- and threo-dihydrosphingosines and sphingosines", & HOPPE-SEYLER'S Z. PHYSIOL. CHEM. 348(12), 1561-9(1967)

CHEMICAL ABSTRACTS, vol. 73, no. 9, 31st August 1970, page 262, abstract no. 44807e, Columbus, Ohio, US; A. CASTEK et al.: "Synthesis of long-chain alkynes", & BULL. SCI., CONS. ACAD. SCI. ARTS RSF YOUGOSLAVIE, SECT. A 1970, 15(5-6), 157

## Description

This invention relates to novel chemical compounds useful *inter alia* as inhibitors of slow reacting substance of anaphylaxis, to compositions containing them, to methods for their preparation and to methods for using said compounds and compositions.

The invention additionally relates to novel pharmaceutical uses of compounds described in US-A-3 217 004 and EP-A-0 169 033. The former document, published 9th November 1965, describes tin salts of carboxymercaptals and their use as stabilizers for solid polymers. Example 11 describes the production of isodecane-1,1-bis (mercaptopropionic acid). US-A-3 217 004 does not suggest any pharmacological uses for the compounds described.

EP-A-0 169 033, published 28th August 1985 and forming part of the state of the art according to Article 54(3) EPC, discloses compounds of structure (I).

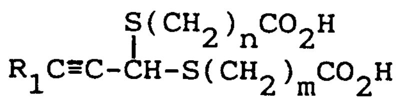

(I)

wherein m and n are independently 1, 2 or 3 and $R_1$ is a straight or branched $C_{8-13}$ alkyl group, or a pharmaceutically acceptable salt thereof, and their use in treating asthma.

The present invention provides a compound having the structural formula A

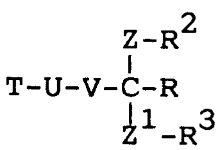

A

wherein T is straight or branched chain alkyl having from 7-15 carbon atoms which may optionally contain from 1-3 non-cumulative double or triple bonds;

U is $-CH_2CH_2-$, $-CH=CH-$ or $-C{\equiv}C-$;

V is straight or branched chain alkylene having from 1 to 4 carbon atoms or is a direct bond;

Z and $Z^1$ are both W-X or both Y or one is W-X and the other is Y, where W is O or $S(O)_m$, [wherein $\underline{m}$ is O, 1 or 2];

X is a straight or branched chain divalent hydrocarbon group having from 2 to 12 carbon atoms which may optionally contain from 1 to 3 non-cumulative double or triple bonds and which may be optionally substituted with the group $-NHR^a$ [wherein $R^a$ is hydrogen, alkyl having from 1 to 6 carbon atoms, $COCF_3$, $CO(CH_2)_2CH(NH_2)CO_2H$, or $SO_2R^b$ (wherein $R^b$ is alkyl having from 1 to 6 carbon atoms or $CF_3$)];

$R^2$ and $R^3$ may be the same or different and are hydrogen, $CH_2OR^c$ [wherein $R^c$ is hydrogen, carboxylic acyl having from 1 to 6 carbon atoms, tetrahydro-2H-pyran-2-yl or $COCH_2CH_2CO_2H$], CHO, 2-tetrazolyl, $COR^d$ [wherein $R^d$ is hydroxy, alkoxy having from 1 to 6 carbon atoms, $OCH_2OC(O)C(CH_3)_3$, $NHR^e$ (wherein $R^e$ is hydrogen, alkyl having from 1 to 6 carbon atoms or $CH_2CO_2H$)] or $SO_3H$, with the proviso that at least one of $R^2$ and $R^3$ is 2-tetrazolyl or carboxyl;

Y is straight or branched chain alkylene having from 1 to 12 carbon atoms which may optionally contain from 1 to 3 non-cumulative double or triple bonds; with the proviso that, when only one group Y is present it may optionally be substituted with the group $OR^c$, where $R^c$ is as defined above;

and

R is hydrogen or, where Z and $Z^1$ are both W-X, R can also be straight or branched chain alkyl having from 1 to 6 carbon atoms, or, where Z and $Z^1$ are both Y, R can also be hydroxyl or fluorine or can be combined with Z and with the mutually bonded carbon atom to form a C-Z double bond or a cycopropane ring;

and the pharmaceutically acceptable salts thereof.

The compounds defined above, pharmaceutical compositions containing them and their pharmaceutical uses are novel, subject to certain provisos.

Specifically,

(1) the compounds of formula A and the pharmaceutically acceptable salts thereof are novel, with the

following provisos:

(A) where R is hydrogen, U is -C≡C-, V is a direct bond, -Z-$R^2$ is -S-$(CH_2)_{2-3}$ COOH and -$Z^1$-$R^3$ is -S-$(CH_2)_{2-3}$COOH, T is other than $C_{8-13}$ alkyl;

(B) said compound of formula A is not isodecane-1, 1-bis(mercaptopropionic acid);

(2) pharmaceutical compositions comprising a compound of formula A or a pharmaceutically acceptable salt thereof are novel with the proviso that where R is hydrogen, U is -C≡C-, V is a direct bond, -Z-$R^2$ is -S-$(CH_2)_{2-3}$ COOH and -$Z^1$-$R^3$ is -S-$(CH_2)_{2-3}$COOH, T is other than $C_{8-13}$ alkyl.

(3) the pharmaceutical use of compounds of formula A in treating allergic reactions or psoriasis or inflammation in a mammal or for reducing the severity of myocardia infarction resulting from heart attack in a mammal are novel, with the proviso that where said use comprises the treatment of allergic reactions and where R is hydrogen, U is -C≡C-, V is a direct bond, -Z-$R^2$ is -S-$(CH_2)_{2-3}$ COOH and -$Z^1$-$R^3$ is -S-$(CH_2)_{2-3}$COOH, T is other than $C_{8-13}$ alkyl.

The divalent hydrocarbon group in X may for example contain an aromatic moiety, such as a benzene ring; e.g. it may be the group -$CH_2$-(p-$C_6H_4$)-. However, X is preferably a straight or branched chain alkylene group.

When R is combined with Z and with the mutually bonded carbon atom to form a cyclopropane ring, R itself forms the unsubstituted methylene group of that ring.

The pharmaceutically acceptable salts may be formed with acids or with bases, according to the presence of basic or acidic functional groups in the molecule. As examples of basic functional groups there may be mentioned -$NHR^a$ when $R^a$ is hydrogen, alkyl having from 1 to 6 carbon atoms or $CO(CH_2)_2CH$-$(NH_2)CO_2H$. As examples of acidic functional groups there may be mentioned -$NHR^a$ when $R^a$ is $CO(CH_2)$-$_2CH(NH_2)CO_2H$ or $SO_2R^b$, $R^2$ and/or $R^3$ when at least one of these is $SO_3H$, 2-tetrazolyl, $CH_2OR^c$ wherein $R^c$ is $COCH_2CH_2CO_2H$, or $COR^d$ wherein $R^d$ is hydroxy or $HNR^e$ provided that $R^e$ is $CH_2CO_2H$.

In the pharmaceutically acceptable salts formed with acids the acid may be for example hydrochloric, sulfuric, phosphoric, citric, acetic, succinnic, methanesulfonic, malic, oxalic, malonic, salicylic, ascorbic, maleic or fumaric acid. The pharmaceutically acceptable salts formed with bases may for example contain (as cation) alkali and alkaline earth metals such as sodium, potassium, magnesium and calcium, or ammonia or substituted amines such as diethanolamine and N-methylglucamine.

Preferred groups of compounds covered by formula A are defined by the following formulae I, II and III:

Compounds of the formula:

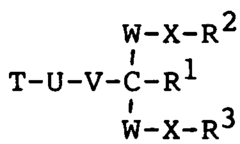

I

wherein T, U, V, W, $R^2$ and $R^3$ are as defined above; each X is a straight or branched chain alkylene having from 2 to 12 carbon atoms which may optionally contain from 1 to 3 non-cumulative double or triple bonds and which may be optionally substituted with the group -$NHR^a$ (wherein $R^a$ is as defined above);
and $R^1$ is hydrogen or a straight or branched chain alkyl having from 1-6 carbon atoms;

Compounds of the formula:

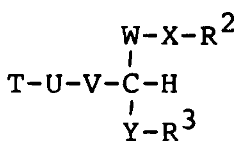

II

wherein T, U, V, W, X, Y, $R^2$ and $R^3$ are defined above; and

Compounds of the formula:

$$\begin{array}{c} \qquad\qquad Y-R^2 \\ \qquad\qquad | \\ T-U-V-C-R^4 \\ \qquad\qquad | \\ \qquad\qquad Y-R^3 \end{array} \qquad\qquad III$$

wherein T, U, V, Y, $R^2$ and $R^3$ are as defined above; and $R^4$ is hydrogen or fluorine or hydroxyl or can be combined with one group Y and with the mutually bonded carbon atom to form a C-Y double bond or a cyclopropane ring;

and the pharmaceutically acceptable salts of the compounds of the formulae I, II and III.

Preferred compounds of formulae I, II and III include those wherein:

T is straight chain alkyl having from 7-15 carbon atoms;

U is -C≡C-;

V is a direct bond;

and $R^2$ and $R^3$ are $CO_2H$;

and their pharmaceutically acceptable salts with bases.

In addition, in formula I

each W is O or S;

each X is a straight or branched chain alkylene having from 2 to 8 (preferably from 2 to 6) carbon atoms;

and $R^1$ is hydrogen;

in formula II

W is O or S;

and X and Y are straight or branched chain alkylene having from 2 to 8, especially from 2 to 6, carbon atoms;

and in formula II

each Y is a straight or branched chain alkylene having from 2 to 8, especially from 2 to 6, carbon atoms;

and $R^4$ is hydrogen or hydroxyl or is combined with one group Y and with the mutually bonded carbon atom to form a double bond.

In two particularly preferred groups of compounds of formula I, II or III, the substituents T-U-V- taken together form the 1-tetradecynyl group,

i.e. n-$C_{12}H_{25}$C≡C-;

and/or the substituents $R^2$ and $R^3$ may be the same or different and are $COR^d$ wherein $R^d$ is as defined above.

Particularly preferred compounds of the invention include:

butanoic acid, 4,4'-[(2-pentadecynylidene)bis(oxy)]bis-;
hexanoic acid, 6,6'-[(2-pentadecynylidene)bis(oxy)]bis-;
(±)-pentanoic acid, 4,4'-[(2-pentadecynylidene)bis(oxy)] bis-;
(±)-heptanoic acid, 6,6'-[(2-pentadecynylidene)bis(oxy)] bis-;
(±)-6-(4-hydroxybutoxy)-5-oxaeicos-7-ynoic acid;
hexanoic acid, 6,6'-[(2-pentadecynylidene)bis(thio)]bis-;
butanoic acid, 4,4'-[(tridecylidene)bis(oxy)]bis-;
butanoic acid, 4,4'-[(pentadecylidene)bis(oxy)]bis-;
(±)-hexanoic acid, 4,4'-[(2-pentadecynylidene)bis(oxy)] bis-;
(±)-6-(carboxyethylthio)-7-eicosynoic acid;
(±)-6-(carboxypropylthio)-7-eicosynoic acid;
(+) and (-)-6-(carboxypentylthio)-7-eicosynoic acid;
(±)-6-(carboxypentyloxy)-7-eicosynoic acid;
(±)-6-[2-(N-trifluoroacetamido)-ethylthio]-7-eicosynoic acid;
(±)-6-(2-amino-3-hydroxy-3-oxopropylthio)-7-eicosynic acid, dipotassium salt;
(±)-6-(3-hydroxy-3-oxo-2-trifluoroacetylamino-propylthio) -7-eicosynoic acid;
6-hydroxy-6-(1-tetradecynyl)-1,11-undecanedioic acid;
6-(1-tetradecynyl)-1,11-undecanedioic acid;
6-(1-tetradecynyl)-1,11-undec-5(E)- and -(Z)ene-dioic acids;
(±)-pentanoic acid, 4,4'-[(2-pentadecynylidene)bis(thio)] bis-;
6-hydroxy-6-(tetradecyl)-1,11-undecanedioic acid;

2-butynoic acid, 4,4'-[(2-pentadecynylidene)bis(oxy)]bis-;
methanesulfonamide, N,N'-[2-pentadecynylidene-bis(oxy-5,1-hexanediyl)]bis-; or
hexanoic acid, 5-[1-(5-amino-1-methyl-5-oxopentyloxy)-2-pentadecynyloxy].

The invention further provides pharmaceutical compositions which comprise a compound of the formula A or a pharmaceutically acceptable salt thereof in association with a pharmaceutically acceptable carrier.

The invention further provides:

(i) a method for treating allergic reactions or psoriasis in a mammal, or

(ii) a method for treating inflammation in a mammal, or

(iii) a method for reducing the severity of myocardial infarction resulting from heart attack in a mammal,

which comprises administering the above-defined pharmaceutical composition or an effective amount of a compound of the formula A or pharmaceutically acceptable salt thereof to said mammal.

The compounds of formula A can be prepared by standard methods. Typical methods are now discussed.

The compounds of the invention having formula I and their pharmaceutically acceptable salts may be prepared by reacting a compound having formula X or XA with a compound having formula XI,

$T\text{-}U\text{-}V\text{-}CR^1(OR'')_2$ X

$T\text{-}U\text{-}V\text{-}COR^1$ XA

$HWXR^2$ XI

wherein T, U, V, W, X, $R^1$ and $R^2$ are as defined herein and R'' is any convenient etherifying group, e.g. an alkyl group, preferably ethyl. This reaction is preferably carried out under conditions whereby the water or alcohol R''OH liberated in the reaction is continuously removed as it is formed. This continuous removal may be accomplished by azeotropic distillation using a solvent such as benzene or toluene. The reaction proceeds best when catalyzed by acid, e.g. p-toluenesulfonic acid. When 2 equivalents or more of reactant XI are utilized, compounds having structural formula I wherein $-W\text{-}X\text{-}R^2$ and $-W\text{-}X\text{-}R^3$ are the same will be produced. When compounds having structural formula I wherein $-W\text{-}X\text{-}R^2$ and $-W\text{-}X\text{-}R^3$ are different are desired, the reaction may be accomplished in two separate steps utilizing one equivalent of the desired reactant, XI, in each step.

A compound of formula I wherein one symbol W is sulfur can be prepared by reaction of a compound of formula I where each W is oxygen with one equivalent of a compound of formula XI wherein W is sulfur to thereby displace either the $W\text{-}X\text{-}R^2$ or the $W\text{-}X\text{-}R^3$ substituent. For purposes of this procedure, the $W\text{-}X\text{-}R^2$ and $W\text{-}X\text{-}R^3$ substituents of starting compound I should ideally be identical. This procedure is conveniently carried out using an acid catalyst such as boron trifluoride.

Compounds having structural formula I wherein at least one symbol W is sulfur may be oxidized to the corresponding sulfoxide or sulfone by known procedures.

Compounds having formula II may be prepared from a compound having formula XII, wherein T, U, V, Y and $R^3$ are as defined above

$T\text{-}U\text{-}V\text{-}CH(OH)YR^3$ XII.

Compounds having formula II wherein W is sulfur may be prepared by first converting the hydroxyl substituent of compound XII to a more readily displaceable substituent such as a hydrocarbon sulfonate ester, e.g. the methane sulfonate ester, XIII,

$T\text{-}U\text{-}V\text{-}CH(OSO_2CH_3)YR^3$ XIII

or to the corresponding bromo or an activated phosphorus substituent.

The conversion of XII to XIII may be carried out by treating XII with methanesulfonyl chloride under standard conditions. Compound XIII may then be treated

$HS\text{-}X\text{-}R^2$ XIV

using known conditions to produce the desired compound having formula II wherein W is sulfur. If desired the compound may thereafter be oxidized at the sulfur atom to the corresponding sulfoxide or sulfone by known procedures.

Compounds having formula II wherein W is oxygen may be prepared from compound XIII in a similar manner by

$HO\text{-}X\text{-}R^2$ XV.

Alternatively, compound XII may be converted into compound II wherein W is oxygen by direct alkylation on the hydroxyl oxygen atom. Thus, for example, XII (in an anhydrous solvent medium) may be treated with a base such as sodium hydride to form the corresponding sodium salt of the alcohol, XVI

$T\text{-}U\text{-}V\text{-}CH(ONa)YR^3$ XVI.

This salt may next be treated with a halide compound $Hal\text{-}X\text{-}R^2$, where Hal is a halogen atom, e.g. bromine or preferably iodine, to produce a compound having formula II wherein W is oxygen.

Compounds having formula III may be prepared by reacting an anion derived from a compound having formula XVII

T-U-V-H XVII

i.e. compound XVIII

$T\text{-}U\text{-}V^{-}\ M^{+}$ XVIII

wherein $M^+$ is one equivalent of a metal cation such as the lithium cation, or an equivalent complex metal cation, e.g. $BrMg^+$, with a carbonyl compound having formula XIX

$R^2\text{-}Y\text{-}CO\text{-}Y\text{-}R^3$ XIX

using known procedures. This reaction will produce compounds having formula III wherein $R^4$ is a hydroxyl group. This tertiary alcohol function may thereafter be converted to other $R^4$ substituents by known methods if desired. For example, treatment of the tertiary alcohol with diethylaminosulfur trifluoride will produce the corresponding compound wherein $R^4$ is fluorine. The tertiary alcohol may be dehydrated to produce a compound wherein $R^4$ and one group Y are combined to form a double bond. This double bond compound may be reduced to produce a compound wherein $R^4$ is hydrogen, or it may be reacted with methylene carbene to produce a corresponding cyclopropyl compound, i.e. a compound having formula III wherein $R^4$ is combined with one group Y and with the mutually bonded carbon atom to form a cyclopropane ring.

In the above-described reactions certain substituents may have to be protected in order to avoid unwanted side-reactions. Thus in particular the $R^2$ and $R^3$ substituents may be protected by methods recognized in the art, especially for example by etherification. In addition, the groups $R^2$ and $R^3$ in a compound of formula A may be modified, if desired, by known procedures. Thus, for example, a compound wherein $R^2$ or $R^3$ is $CH_2OH$ may be converted to a compound wherein $R^2$ or $R^3$ is $CO_2H$ by oxidation or to a compound wherein $R^2$ or $R^3$ is $CH_2OCOCH_3$ by acylation.

For purposes of completeness, the following abbreviated reaction sequence is given to exemplify a process for performing selective reactions where multiple sites of unsaturation are present. Other such sequences are known in the art.

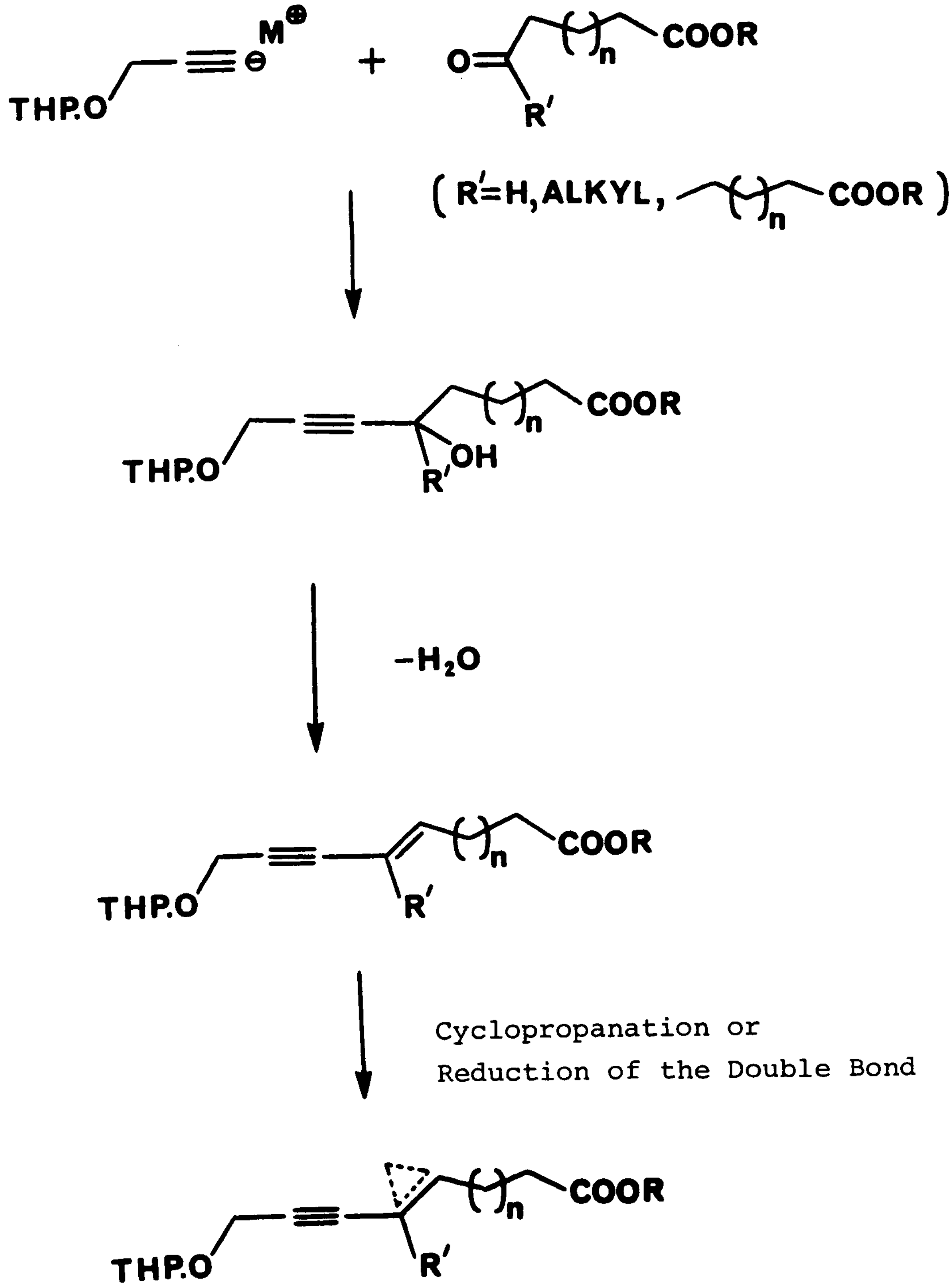
( R'=H, ALKYL, COOR )
−$H_2O$
Cyclopropanation or
Reduction of the Double Bond

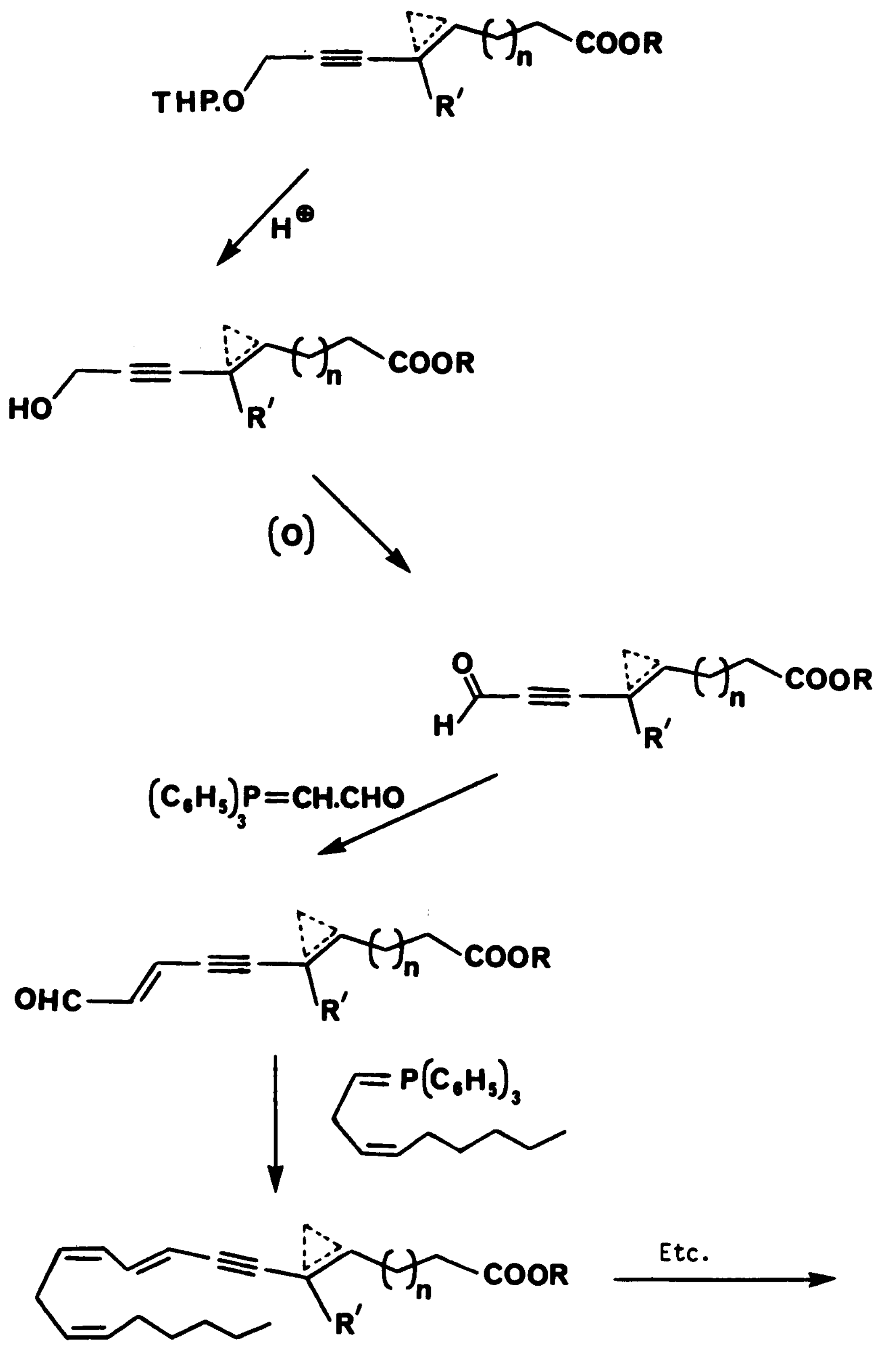


In the above abbreviated reaction sequence, THP indicates the 2-tetrahydro-2H-pyranyl radical. Double bonds can be regiospecifically included or excluded by proper choice of carbon-to-carbon bond-forming reactions and reagents which will be known to those skilled in the art.

The above-described starting materials are either known compounds or preparable from known compounds by methods known in the art.

Thus, compound X is an acetal or ketal, preparable from the corresponding aldehyde or ketone by well known methods. In an alternative method, a compound such as XX may be reacted with an orthoester such as ethyl orthoformate by known methods to produce the acetal XXI

$C_{12}H_{25}C{\equiv}CH + HC(OEt)_3$ XX

$C_{12}H_{25}C{\equiv}CCH(OEt)_2$ XXI.

Compounds having formula XII are secondary alcohols which may be prepared, for example, by the reaction of a Grignard reagent such as XXII with an aldehyde such as XXIII

T-U-V-MgBr XXII

$OCHYR^3$ XXIII.

Known equivalent reactants to XXII such as lithium reagents, e.g. XXIV, may also be utilized

$C_{12}H_{25}C{\equiv}CLi$ XXIV.

Salts of compounds of the invention with inorganic or organic bases can be prepared for example by reaction with sodium hydroxide, sodium carbonate, sodium bicarbonate, potassium hydroxide, calcium hydroxide, ammonia and amines. These salts may be converted back to their respective acids by treatment with an acid such as dilute hydrochloric acid. The acids and their respective salts differ in certain physical properties such as solubility but are otherwise equivalent for purposes of the invention.

Salts of compounds of the invention with organic or inorganic acids can be prepared by contacting the free base with a sufficient amount of the desired acid to produce a salt in the conventional manner. The free base may be regenerated by treating the salt with a base, for example, dilute aqueous base such as sodium hydroxide, potassium carbonate, ammonia, and sodium bicarbonate solutions. The free bases differ from their respective salts somewhat in certain physical properties such as solubility, but the salts are otherwise equivalent to their respective free bases for purposes of the invention.

The compounds of the invention may exist in unsolvated as well as solvated forms, including hydrated forms. In general, these solvated forms with pharmaceutically acceptable solvents such as water or ethanol are equivalent to the unsolvated forms for purposes of the invention.

Certain compounds of the invention may exist in isomeric forms. The invention includes all such isomers both in pure form and in admixture, including racemic mixtures.

The compounds of this invention can be used to treat allergy-caused diseases and psoriasis, but their preferred use is for treating allergic chronic obstructive lung diseases. In chronic obstructive lung disease, as used herein, the passage of air through the lungs is obstructed or diminished as in asthma, bronchitis and the like.

The anti-allergy effects of the compounds of this invention may be identified by tests which measure a compound's inhibition of leukotriene-$C_4$-induced contraction of lung smooth muscle. The substance leukotriene-$C_4$ is a component of slow reacting substance of anaphylaxis (SRS-A). For example, the compound hexanoic acid, 6,6'-[(2-pentadecynylidene)bis(oxy)]bis-, was found to inhibit leukotriene-$C_4$-induced contractions of lung smooth muscle in such a test procedure <u>in vitro</u> at a concentration of $10^{-5}$ Molar. This compound was also found to inhibit leukotriene-$C_4$-induced bronchospasm in guinea pigs <u>in vivo</u> at an intratracheal dose of 0.5 mg/kg or intravenous dose of 10 mg/kg.

<u>Measurement of inhibition of leukotriene-$C_4$-induced contractions in vitro</u>:

A guinea pig is killed and the lung is removed; the trachea, bronchi and large blood vessels are discarded. Strips of lung parenchyma are prepared from the lower lobes of the lung. The strips are suspended in a heated organ bath containing 10 ml of oxygenated Tyrodes solution. Isometric tension is measured. A contractile response to $10^{-8}$ Molar leukotriene-$C_4$ is generated in the absence and presence of test compound and the percent inhibition produced by the test compound is calculated.

<u>Measurement of inhibition of leukotriene-$C_4$-induced bronchospasm in guinea pigs in vivo</u>:

Fasted male guinea pigs are anesthetized with dialurethane i.p. (0.9 ml/kg of a solution containing

diallylbarbituric acid 0.1 g/ml, ethylurea 0.4 g/ml and ethyl carbamate 0.4 g/ml) and prepared for the measurement of intratracheal pressure as modified from H. Konzett and R. Rossler, Nauyn-Schmeidebergs Arch. Exp. Path. Pharmakol. 195: 71-74, 1940. A bronchospasm [as measured by the increase in intratracheal pressure] is induced by the intratracheal administration of 0.3 µg of leukotriene-$C_4$ delivered in 0.1 ml of isotonic saline solution. The test compound is administered either 10 min intravenously or 5 min intratracheally before the administration of leukotriene-$C_4$. The bronchospasm to leukotriene-$C_4$ is measured and a percent inhibition by the test compound is calculated.

When administered orally the compounds of the invention are active at doses from about 10 to 500 mg/kg of body weight; when administered parenterally, e.g., intravenously, the compounds are active at dosages from about 0.1 to 10 mg/kg body weight; when administered by inhalation (aerosol or nebulizer) the compounds are active at dosages of about 0.1 to 5 mg per puff, and one to four puffs may be taken every 4 hours.

The compounds of this invention are also useful for the treatment of inflammation. The anti-inflammatory use of the compounds of the present invention may be demonstrated by the Reversed Passive Arthus Reaction (RPAR) Synovitis technique as set forth below using male Lewis rats (obtained from Charles River Breeding Laboratories) weighing 200-250 grams. The potency of the compounds is determined using indomethacin as the standard. On the basis of the test results, an oral dosage range of about 10 mg/kg/day to about 500 mg/kg/day in divided doses taken at about 4 hour intervals is recommended.

RPAR Synovitis Technique

A Lewis rat is dosed orally with drug or placebo one hour prior to intravenous administration of 2.28 mg of bovine serum albumin (BSA) in 0.2 cc of pyrogen-free saline followed by the intra-articular injection of 0.54 mg of rabbit anti-BSA antibody in 0.03 cc of pyrogen-free saline into one knee joint. The other knee is injected with 0.03 cc of pyrogen-free saline. All injections are made with the animal under light ether anesthesia. Three hours later the rat is again dosed orally with drug or placebo. All drug doses are split. That is, one-half of the dose is administered before lesion induction and one-half is administered after lesion induction.

The following morning (about 17 hours after lesion induction) the rat is killed and both knee joints are exposed. The subpatellar areolar tissue with attendant synovium is excised and weighed. Differences between the weight of antibody- and saline-injected knees are considered to represent the inflammatory response for each animal (delta synovial weight). Differences in delta synovial weight between lesion controls and drug-treated rats are evaluated for statistical significance with an analysis of variance. Relative potencies are determined with a linear regression analysis.

For preparing pharmaceutical compositions from the compounds described by this invention, inert, pharma-ceutically acceptable carriers can be either solid or liquid. Solid form preparations include powders, tablets, dispersible granules, capsules, cachets and suppositories. A solid carrier can be one or more substances which may also act as diluents, flavoring agents, solubilizers, lubricants, suspending agents, binders or tablet disintegrating agents; it can also be an encapsulating material. In powders, the carrier is a finely divided solid which is in admixture with the finely divided active compound. In the tablet the active compound is mixed with carrier having the necessary binding properties in suitable proportions and compacted into the shape and size desired. The powders and tablets preferably contain from 5 or 10 to about 70 percent of the active ingredient. Suitable solid carriers are magnesium carbonate, magnesium stearate, talc, sugar, lactose, pectin, dextrin, starch, gelatin, tragacanth, methylcellulose, sodium carboxymethylcellulose, a low melting wax, cocoa butter and the like. The term "per-paration" is intended to include the formulation of the active compound with encapsulating material as carrier providing a capsule in which the active component (with or without other carriers) is surrounded by carrier, which is thus in association with it. Similarly, cachets are included. Tablets, powders, cachets and capsules can be used as solid dosage forms suitable for oral administration.

For preparing suppositories, a low melting wax such as a mixture of fatty acid glycerides or cocoa butter is first melted, and the active ingredient is dispersed homogeneously therein as by stirring. The molten homogeneous mixture is then poured into convenient sized molds and allowed to cool and thereby solidify.

Liquid form preparations include solutions, suspensions and emulsions. As an example may be mentioned water or water-propylene glycol solutions for parenteral injection. Liquid preparations can also be formulated in solution in aqueous polyethylene glycol solution. Aqueous solutions suitable for oral use can be prepared by adding the active component in water and adding suitable flavors and coloring, stabilizing, sweetening, solubilizing and thickening agents as desired. Aqueous suspensions suitable for oral use can be

made by dispersing the finely divided active component in water with viscous material, i.e., natural or synthetic gums, resins, methylcellulose, sodium carboxymethylcellulose and other well-known suspending agents.

Also included are solid form preparations which are intended for conversion, shortly before use, into liquid form preparations for either oral or parenteral administration. Such liquid forms include solutions, suspensions and emulsions. These particular solid form preparations are most conveniently provided in unit dose form and as such are used to provide a single liquid dosage unit. Alternately, sufficient solid may be provided so that after conversion to liquid form, multiple individual liquid doses may be obtained by measuring predetermined volumes of the liquid form pre-paration as with a syringe, teaspoon or other volumetric container. When multiple liquid doses are so prepared, it is preferred to maintain the unused portion of said liquid doses at low temperature (i.e., under refrigera-tion) in order to retard possible decomposition. The solid form preparations intended for conversion into liquid form may contain, in addition to the active material, flavorings, colorings, stabilizers, buffers, thickeners, artificial and natural sweeteners, dispersing and solubilizing agents and the like. The solvent utilized for preparing the liquid form preparation may be water, isotonic water, ethanol, glycerine, propylene glycol and the like as well as mixtures thereof. Naturally, the solvent utilized will be chosen with regard to the route of administration; for example, liquid preparations containing large amounts of ethanol are not suitable for parenteral use.

Preferably, the pharmaceutical preparation is in unit dosage form. In such form, the preparation is subdivided into unit doses containing appropriate quantities of the active component. The unit dosage form can be a packaged preparation, the package containing discrete quantities of preparation, for example, packeted tablets, capsules and powders in vials or ampoules. The unit dosage form can also be a capsule, cachet or tablet itself or it can be the appropriate number of any of these in packaged form.

The quantity of active compound in a unit dose of preparation may be varied or adjusted from 1 mg to 100 mg according to the particular application and the potency of the active ingredient. The compositions can, if desired, also contain other therapeutic agents.

The dosages may be varied depending upon the requirements of the patient, the severity of the condition being treated and the particular compound being employed. Determination of the proper dosage for a particular situation is within the skill of the art. Generally, treatment is initiated with smaller dosages which are less than the optimum dose of the compound. Thereafter, the dosage is increased by small increments until the optimum effect under the circumstances is reached. For convenience, the total daily dosage may be divided and administered in portions during the day if desired.

The following Examples illustrate the invention:

## EXAMPLE 1

### 2-PENTADECYNYL ALDEHYDE DITHIOACETAL OF N-TRIFLUOROACETYL CYSTEINE METHYL ESTER

A solution of the 2-pentadecynyl aldehyde (0.3098 g) in dry $CH_2Cl_2$ (5 ml) was treated with N-trifluoroacetyl cysteine methylester, freshly prepared by zinc reduction of the corresponding disulfide (1 g; required 0.9667 g), followed by trimethylsilyl chloride (0.15 g). The reaction was stirred at room temperature for 1 hour. Evaporation of $CH_2Cl_2$ in vacuo gave a gummy residue which was distributed between $CH_2Cl_2$ and water. The $CH_2Cl_2$ phase was separated, and the aqueous phase extracted once with $CH_2Cl_2$. The combined $CH_2Cl_2$ extracts were dried over $Na_2SO_4$ and evaporated to dryness to provide the crude product which was purified on a coarse silica gel (30 g) column using 20-50% diethyl ether in n-hexane. Yield: 0.6893 g.

## EXAMPLE 2

### 1-BUTANOL, 4,4'-[(2-PENTADECYNYLIDENE)BIS(OXY)]BIS-, DIBENZOATE

A mixture of 2-pentadecynal diethylacetal (5.0 g), butane-1,4-diol monobenzoate (6.5 g) and p-toluenesulfonic acid (0.2 g) in dry benzene was refluxed with azeotropic removal of ethanol. After 3 hours the dark reaction solution was washed with aqueous $NaHCO_3$, water, then brine. The solvent was removed in vacuo to give crude product (11.0 g) as a brownish oil which was used as such in the next reaction.

## EXAMPLE 3

### 1-BUTANOL, 4,4'-[(2-PENTADECYNYLIDENE)BIS(OXY)]BIS-

The product from Example 2 (4.0 g) was hydrolyzed with 30% KOH in aqueous ethanol (120 ml) by refluxing the reaction mixture for 2 hours. Ethanol was evaporated under vacuum and the residue was washed with water. The product was extracted with diethyl ether, dried over $MgSO_4$, filtered and evaporated to dryness in vacuo to give 1.8 g of the product as a yellowish oil.

EXAMPLE 4

BUTANOIC ACID, 4,4'-[(2-PENTADECYNYLIDENE)BIS(OXY)]BIS-, DIMETHYLESTER

A. The acetal-diol (1 g) from Example 3 was dissolved in dry DMF (20 ml) and treated with pyridinium dichromate (8.2 g). The mixture was stirred at room temperature for 20 hours, diluted with 200 ml water and extracted with approximately 400 ml diethylether in portions. Drying over $Na_2SO_4$ and evaporation of the ether extract gave the crude diacid.

The reaction was repeated with acetal-diol (0.488 g) in 10 ml DMF and 4.1 g pyridinium dichromate. Work-up as above gave the crude diacid.

B. The two products from Part A were combined and treated with diazomethane (from 6.5 g p-tolylsulfonylmethylnitrosamide). Excess diazomethane was removed by carefully bubbling nitrogen through the solution. Evaporation of diethylether gave the crude diester which was chromatographed on TLC grade silica gel (50 g) using 5% acetone in n-hexane as eluent. The pure dimethylester (0.6813 g) was obtained as a yellowish oil.

EXAMPLE 5

BUTANOIC ACID, 4,4'-[(2-PENTADECYNYLIDENE)BIS(OXY)]BIS-

The dimethylester (0.5 g) from Example 4 was dissolved in ethanol (10 ml) and treated with 10% aqueous NaOH (5 ml). After stirring at room temperature for 36 hours, ethanol was evaporated off in vacuo, the residue taken up in water and extracted once with diethylether. The remaining aqueous phase was acidified to pH 1.5 with aqueous oxalic acid and extracted with $CH_2Cl_2$. Drying the $CH_2Cl_2$ extract over $Na_2SO_4$ followed by evaporation in vacuo provided the pure diacid as a colorless crystalline solid, m.p. 62-63 °C. Yield: 0.4496 g.

EXAMPLE 6

1-BUTANOL, 4,4'-[(2-NONYNYLIDENE)BIS(OXY)]BIS-, DIBENZOATE

A mixture of 2-nonynal diethylacetal (4.4 g), butane-1,4-diol monobenzoate (8.2 g) and a catalytic amount of p-toluenesulfonic acid in dry benzene was refluxed for 3 hours as in Example 2. Work-up as in Example 2 gave the crude product which was purified by passing through a $SiO_2$ column and eluting with hexane and chloroform (1:1) to give 9.0 g of the product.

EXAMPLE 7

1-BUTANOL, 4,4'-[(2-NONYNYLIDENE)BIS(OXY)]BIS-

The product from Example 6 (1.0 g) was hydrolyzed with 30% KOH in aqueous ethanol as in Example 3. The product so obtained was purified by preparative TLC using 5% methanol in $CHCl_3$ as eluent. Yield: 0.3 g.

EXAMPLE 8

BUTANOIC ACID, 4,4'-[(2-NONYNYLIDENE)BIS(OXY)]BIS-

The product from Example 7 (O.5 g) was oxidized with pyridinium dichromate (4.1 g) in 13 ml of dry DMF at room temperature for 2 days. The mixture was diluted with water (350 ml) and extracted with diethylether (3 x 100 ml); the extract was washed with water, dried ($Na_2SO_4$) and filtered. The solvent was evaporated to give 0.4 g crude diacid, which was purified by methylation with diazomethane, chromatography to give pure dimethylester, and hydrolysis as in Example 5 to give 0.3 g pure diacid.

EXAMPLE 9

BENZENEMETHANOL, 4,4'-[(2-PENTADECYNYLIDENE)BIS(OXY)METHYL]BIS-

Benzene-methanol, 4,4'-[(2-pentadecynylidene)bis(oxy)methyl]bis-, dibenzoate ester was prepared according to Example 2 from 2-pentadecynal and benzenemethanol, 4-benzoyloxymethyl-. The resulting crude reaction mixture was treated with 100 ml of water, 20 g of KOH and 300 ml of absolute ethanol. After stirring the mixture at room temperature overnight, it was refluxed for one hour. The solvents were then evaporated, the oily residue was treated with $CH_2Cl_2$, washed with water, and dried over $Na_2SO_4$. After evaporation of the solvent, the mixture was purified via silica gel chromatography (50% EtOAc/hexanes). Yield: 8.5 g.

EXAMPLE 10

BENZOIC ACID, 4,4'-[(2-PENTADECYNYLIDENE)BIS(OXY)METHYL]BIS-

A. The title compound was prepared in a crude state by oxidation of the product of Example 9 according to Example 4A. For purification, it was converted first into the pure dimethyl ester according to Example 4B.

B. 1.7 g of benzoic acid, 4,4'-[(2-pentadecynylidene)-bis(oxy)methyl]bis-, dimethyl ester were stirred at room temperature in 30 ml of absolute EtOH and 20 ml of 10% aqueous KOH. After two days, the solvents were evaporated under reduced pressure. The residue was taken up in water and acidified with aqueous oxalic acid. The precipitate obtained was filtered off, washed with water and dried. Yield: 1.6 g.

EXAMPLE 11

A. METHYL [4-(METHOXYCARBONYLMETHOXY)]-3-OXAOCTADEC-5-YNOATE

B. METHYL [4-(2-HYDROXYETHOXY)]-3-OXAOCTADEC-5-YNOATE

2.0 g of 4-(2-hydroxyethoxy)-3-oxaoctadecen-5-ynol (Example 31) in 30 ml dry DMF was added in portions to a solution of pyridinium dichromate in 70 ml of dry DMF. After stirring at room temperature for two days, the mixture was poured into water (500 ml). The aqueous solution was extracted several times with $Et_2O$. The combined extracts were washed with water and dried over $Na_2SO_4$. The crude acid resulting from the evaporation of the solvent was treated in $Et_2O$ at 0° C with a large excess of diazomethane solution in $Et_2O$. The esterified mixture (2.0 g) was purified by chromatography on TLC grade silica gel:

Fractions 23-28 gave compound (A), 0.13 g

Fractions 75-85 gave compound (B), 0.11 g.

EXAMPLE 12

PENTANOIC ACID, 5,5'-[(2-PENTADECYNYLIDENE)-BIS(OXY)] BIS-, DIMETHYLESTER

A dry DMF solution (50 ml) of 1-pentanol, 5,5'-[(2-pentadecynylidene)-bis(oxy)]bis- (Example 14) (3.67 g) was added at room temperature to a solution of pyridinium dichromate in 120 ml of dry DMF. After two days the reaction was worked up as in Example 11. The crude reaction mixture was treated at 0° C in $Et_2O$ with a large excess of $CH_2N_2$. The excess diazomethane was destroyed with glacial acetic acid and the reaction mixture was purified by silica gel chromatography (10% EtOAc/Hexanes). Yield: 0.9 g.

EXAMPLE 13

PENTANOIC ACID, 5,5'-[(2-PENTADECYNYLIDENE)-BIS(OXY)] BIS-

0.7 g of pentanoic acid, 5,5'-[(2-pentadecynylidene)-bis(oxy)]bis-, dimethyl ester (Example 12) was stirred at room temperature for 24 hours with 10 ml of absolute EtOH and 10 ml of 10% aqueous KOH. The solvents were then evaporated under reduced pressure. The residue was taken up in water, acidified with aqueous oxalic acid, and extracted several times with $CH_2Cl_2$. The combined extracts were washed with water and dried over $Na_2SO_4$. Evaporation of the solvent yielded 0.65 g. of the title compound.

EXAMPLE 14

1-PENTANOL, 5,5'-[(2-PENTADECYNYLIDENE)-BIS(OXY)]BIS-

A. 1-Pentanol, 5,5'-[(2-pentadecynylidene)bis(oxy)]bis-, dibenzoate ester, was prepared according to Example 2 from 2-pentadecynal diethylacetal and pentane-1,5-diol monobenzoate.

B. The crude reaction mixture from step A was stirred overnight with 150 ml of water and 300 ml of absolute EtOH containing 25 g of KOH. After refluxing for three hours, the solvents were evaporated under reduced pressure. The residue was treated with $CH_2Cl_2$, washed with water and dried over $Na_2SO_4$. The reaction mixture was purified by silica gel chromatography (50% EtOAc/hexanes). Yield: 4.0 g.

EXAMPLE 15

HEXANOIC ACID, 6,6'-[(2-PENTADECYNYLIDENE)-BIS(OXY)]BIS-

3.5 g of 1-hexanol, 6,6'-[(2-pentadecynylidene)-bis(oxy)]bis- (Example 16) in 20 ml of dry DMF was added in portions to a solution of pyridinium dichromate in 70 ml of dry DMF at room temperature. After 24 hours, the reaction was worked up as Example 11. The reaction mixture was purified by silica gel chromatography (first $CHCl_3$; then 5% $MeOH/CHCl_3$). Yield: 70 mg.

EXAMPLE 16

1-HEXANOL, 6,6'-[(2-PENTADECYNYLIDENE)-BIS(OXY)]BIS-

The title compound was prepared and purified in similar manner as in Example 14. The starting material was a mixture containing 1-hexanol, 6,6'-[(2-pentadecynylidene)-bis(oxy)]bis-, dibenzoate ester, prepared according to Example 2 from 2-pentadecynal diethylacetal and hexane-1,6-diol monobenzoate.

EXAMPLE 17

(±)-1-BENZOYLOXY-6-(4-HYDROXYBUTOXY)-5-OXAEICOS-7-YNE

3.5 g of benzoyl chloride in 27 ml of $CH_2Cl_2$ was added dropwise, over a period of three hours, at 0 °C to a solution of 1-butanol, 4,4'-[2-pentadecynylidene-bis(oxy)]bis- (8.0 g) and dry pyridine (15 ml) in 60 ml of $CH_2Cl_2$. After the addition, the mixture was allowed to warm to room temperature and stirred overnight. The mixture was then diluted with $CH_2Cl_2$ (400 ml), washed with water, and dried over $Na_2SO_4$. After evaporation of the solvent, the reaction mixture (10 g) was purified by silica gel chromatography (10% acetone/$CHCl_3$). This material was chromatographed again using $CHCl_3$ as eluent. Yield 4.2 g.

EXAMPLE 18

(±)-6-(4-HYDROXYBUTOXY)-5-OXAEICOS-7-YNOIC ACID

2.2 g of product from Example 17 was oxidized and purified as in Example 15 (eluent: first $CHCl_3$, then 1% $MeOH/CHCl_3$). The product of the oxidation (1.1 g) was hydrolysed and the reaction was worked up as in Example 14. The reaction product was purified by column chromatography ($SiO_2$; eluent: first $CHCl_3$; then 50% $CH_3CN$)/$CHCl_3$; then 30% $MeOH/CHCl_3$). The product obtained from the elution with 30% $MeOH/CHCl_3$ was purified again ($SiO_2$; 5% $MeOH/CHCl_3$) using the same technique. Yield: 0.16 g.

EXAMPLE 19

METHYL 6-[3-(METHOXYCARBONYL)PROPOXY]-5-OXAEICOSANOATE

1.7 g of butanoic acid, 4,4'-[(2-pentadecynylidene)-bis(oxy)]bis-, dimethylester was dissolved in olefin-free petroleum ether (60 ml) and hydrogenated in the presence of 10% Pd/C (0.7 g). After absorption of $H_2$ was complete (3 hours), the catalyst was filtered through celite and washed with $CH_2Cl_2$. The reaction product (1.6 g) obtained after evaporation of the combined filtrates was purified by silica gel chromatog-

raphy (1% acetone in $CHCl_3$). Yield: 0.8 g.

EXAMPLE 20

6-(3-CARBOXYPROPOXY)-5-OXAEICOSANOIC ACID

0.65 g of the product from Example 19 was hydrolyzed in 12 ml of absolute EtOH and 8.1 ml of 10% aqueous KOH as in Example 13 to provide the pure diacid.

EXAMPLE 21

(±)-1-(BENZOYLOXY)-6-ETHOXY-5-OXAEICOS-7-YNE

32 g of ethane, 2,2'-[(2-pentadecynylidene)bis(oxy)]bis-, 25 g of 4-benzoyloxy-1-butanol, and 100 mg of p-toluene sulfonic acid were refluxed in a dry apparatus in 300 ml of benzene using a Dean-Stark trap. After evaporating off 75 ml of solvent, the solution was cooled to room temperature diluted with $CHCl_3$, washed with saturated aqueous $NaHCO_3$, then with water and dried over $Na_2SO_4$. The oil (13 g) resulting after evaporation of the solvent was purified by column chromatography ($SiO_2$) ($CHCl_3$). The partially purified compound was rechromatographed ($SiO_2$) (50% hexanes/$CHCl_3$). Yield: 1.5 g.

EXAMPLE 22

(±)-6-ETHOXY-5-OXAEICOS-7-YN-1-OL

(±)-1-Benzoyloxy-6-ethoxy-5-oxaeicos-7-yne was hydrolyzed using the same procedure as described for Example 14. The reaction mixture was purified in the same manner using $CHCl_3$ as eluent.

EXAMPLE 23

(±)-6-ETHOXY-5-OXAEICOS-7-YNOIC ACID

1.2 g of the product from Example 20 in 15 ml of dry DMF was added in portions to a solution of pyridinium dichromate (4.4 g) in 30 ml of dry DMF. The solution was stirred at room temperature for 24 hours, then poured into water (300 ml). The aqueous mixture was extracted several times with $Et_2O$, the combined extracts were washed with water and dried over $Na_2SO_4$. The reaction mixture was purified by column chromatography ($SiO_2$) (1% MeOH/$CHCl_3$). The partially purified product (0.54 g) was further purified by preparative thin layer chromatography ($SiO_2$) (2% MeOH/$CHCl_3$). Yield: 0.35 g.

EXAMPLE 24

4-METHYL-6-(4-HYDROXY-1-METHYLBUTOXY)-5-OXAEICOS-7-YN-1-OL

The crude mixture containing 1-pentanol, 4,4'-[(2-pentadecynylidene)-bis(oxy)]bis-, dibenzoate ester (prepared according to Example 2 from 2-pentadecynal diethylacetal and pentane-1,4-diol, 1-monobenzoate) was hydrolyzed and purified using the same procedure as described for Example 14 to provide the title compound.

EXAMPLE 25

PENTANOIC ACID, 4,4'-[(2-PENTADECYNYLIDENE)-BIS(OXY)]BIS-

2.6 g of pentanoic acid, 4,4'-[(2-pentadecynylidene)-bis(oxy)]bis-, dimethyl ester was hydrolyzed using the same procedure as described in Example 13. Yield: 2.4 g. The starting material was prepared from the product of Example 24 by the processes of Examples 3 and 4.

EXAMPLE 26

5-METHYL-7-(5-HYDROXY-1-METHYLPENTYLOXY)-6-OXAHENEICOS-8-YN-1-OL

The mixture containing 5-methyl-7-(5-hydroxy-1-methylpentyloxy)-6-oxaheneicos-8-yn-1-ol dibenzoate ester (prepared according to Example 2 from 2-pentadecynal diethylacetal and hexane-1,5-diol, 1-monobenzoate) was hydrolyzed and purified using the same procedure as described in Example 14.

EXAMPLE 27

METHYL [5-METHYL-7-(5-METHOXY-1-METHYL-5-OXOPENTYLOXY)]-6-OXAHENEICOS-8-YNOATE

The product from Example 26 was oxidized, methylated and purified as described in Example 12.

EXAMPLE 28

5-METHYL-7-(4-CARBOXY-1-METHYLBUTOXY)-6-OXAHENEICOS-8-YNOIC ACID

The product from Example 27 was hydrolyzed using the same procedure as described for Example 13.

EXAMPLE 29

METHYL CIS-6-(4-METHOXY-4-OXOBUTOXY)-5-OXAEICOS-7-ENOATE

0.4 g of Lindlar catalyst in petroleum ether (10 ml; olefin- and sulfur-free) containing 5 drops of a 5% solution of quinoline in petroleum ether was equilibrated under $H_2$ at room temperature and atmospheric pressure. After four hours, 1.0 g of butanoic acid, 4,4'-[(2-pentadecynylidene)-bis(oxy)]bis-, dimethyl ester in 20 ml of petroleum ether (olefin- and sulfur-free) was added. The mixture absorbed 47 ml of $H_2$ (theory: 55 ml). The catalyst was then removed by filtration through "Celite" and washed several times with petroleum ether. Evaporation of the solvent yielded 0.9 g of the title compound.

EXAMPLE 30

CIS-6-(3-CARBOXYPROPOXY-5-OXAEICOS-7-ENOIC ACID, DIPOTASSIUM SALT

0.6 g of product from Example 29 was dissolved in 15 ml of absolute EtOH and 8 ml of 10% aqueous KOH was added. After stirring at room temperature for 24 hours, the solvents were evaporated and the residue was applied as water solution to 65 g of XAD-4 resin column. The column was eluted first with water, then with MeOH. The fractions containing the compound were combined and treated twice with 30% $Et_2O$/hexanes. The solvents were decanted and the remaining product dried in vacuo to provide the title compound.

EXAMPLE 31

4-(2-HYDROXYETHOXY)-3-OXAOCTADEC-5-YN-1-OL

The reaction mixture containing ethanol, 2,2'-[pentadecyn-1-ylidene-bis(oxy)]bis-, dibenzoate ester was hydrolyzed and purified using the same procedure as described in Example 14.

EXAMPLE 32

4-(CARBOXYMETHOXY)-3-OXAOCTADEC-5-YNOIC ACID

0.13 g of methyl 4-[(methoxycarbonyl)methoxy]-3-oxaoctadec-5-ynoate (Example 11) was hydrolyzed using the same procedure described in Example 13. Yield: 0.13 g.

EXAMPLE 33

L-CYSTEINE, S[R or S]-[1-(4-METHOXY-4-OXOBUTOXY)-2-NONYNYL]-N-(TRIFLUOROACETYL)-, METHYLESTER - (LESS POLAR ISOMER) AND L-CYSTEINE, S[R or S]-[1-(4-METHOXY-4-OXOBUTOXY)-2-NONYNYL]-N-(TRIFLUOROACETYL)-, METHYLESTER - (MORE POLAR ISOMER)

A solution of butanoic acid, 4,4'-[(2-nonynylidene)bis(oxy)]bis-, dimethyl ester (pure intermediate from Example 8) (0.6627 g) in dry $CH_2Cl_2$ (5 ml) was treated with freshly prepared N-trifluoroacetyl cysteine methylester (from 0.4300 g corresponding disulfide) and the mixture cooled (dry ice/cyclohexanone bath). With stirring and under an atmosphere of $N_2$, $BF_3.Et_2O$ (0.05 ml) was syringed in. After 1 hour the reaction flask was stirred outside the cooling bath for about 3 minutes and then quenched with dilute (about 7%) $NH_4OH$ solution. Extractive isolation with $CH_2Cl_2$ gave a gummy product (0.9797 g) which was chromatographed on TLC grade silica gel (50 g) using 10% acetone/n-hexane as eluent. Fractions (5 ml each):

41-51 Pure less polar isomer (0.1287 g)

61-81 Pure more polar isomer (0.0831 g) Both isomers were obtained as thick oils.

## EXAMPLE 34

### L-CYSTEINE, S[R or S]-[1-(4-METHOXY-4-OXOBUTOXY)-2-PENTADECYNYL]-N-(TRIFLUOROACETYL)-, METHYLESTER - (LESS POLAR ISOMER) AND L-CYSTEINE, S[R or S]-[1-(4-METHOXY-4-OXOBUTOXY)-2-PENTADECYNYL]-N-(TRIFLUOROACETYL)-,METHYLESTER - (MORE POLAR ISOMER)

A stirred solution of the acetal-diester (1 g; from Example 4) dissolved in dry $CH_2Cl_2$ (6 ml) was treated with cysteine methylester N-trifluoroacetamide (freshly prepared from 0.53 g corresponding disulfide). The mixture was cooled (cyclohexanone/dry ice bath) and the reaction flask was taken out of the cooling bath and stirred for 3-4 minutes followed by treatment with dilute (about 7%) $NH_4OH$. Extractive isolation with $CH_2Cl_2$ gave a gummy product (1.4618 g) which was chromatographed on TLC grade silica gel (75 g) using 10% acetone/n-hexane as eluent. Fractions (5 ml each):

50-58 Pure less polar isomer (0.3783 g)

64-78 Pure more polar isomer (0.2626 g)

Both isomers were obtained as thick oils.

## EXAMPLE 35

### L-CYSTEINE, S[R or S]-[1-(3-CARBOXYPROPOXY)-2-PENTADECYNYL]-, DIPOTASSIUM SALT (FROM LESS POLAR DIASTEREOISOMER)

The dimethylester (0.3783 g; from Example 34, less polar isomer) was subjected to hydrolysis with 0.13 M $K_2CO_3$ (100 ml) in MeOH:water (3:1) at room temperature. After about 36 hours solvents were removed *in vacuo* and the crude dipotassium salt so obtained purified on XAD-4 (140 g) column:

Fraction 1, 600 ml - water (discarded)

Fractions 2-5, 400 ml each - methanol

Evaporation *in vacuo* provided the product as an amorphous solid, 0.1043 g.

## EXAMPLE 36

### L-CYSTEINE, S[R or S]-[1-(3-CARBOXYPROPOXY)-2-PENTADECYNYL]-, DIPOTASSIUM SALT (FROM MORE POLAR DIASTEREOISOMER)

The dimethylester (0.3426 g; from Example 34, more polar isomer) was subjected to hydrolysis with 0.13 M $K_2CO_3$ (100 ml) in MeOH:water (3:1) as in Example 35. Work-up and purification on XAD-4 column (140 g) provided in fractions 2-5 (400 ml each, MeOH) the product (0.1049 g) as a amorphous solid.

## EXAMPLE 37

### PROPANOIC ACID, 4,4'-[4E,6Z,9Z-PENTADECATRIEN-2-YNYLIDENE-BIS(THIO)]BIS

A solution of the product from preparative Example IV (1.0 g) and γ-mercaptobutyric acid (1.1 g) in 24 ml of dry $CH_2Cl_2$ was cooled to -22 °C ($CCl_4/CO_2$) (under $N_2$). To this was added $BF_3.Et_2O$ (0.5 ml). The reaction mixture was stirred at this temperature for 2 hours. The mixture was diluted with $CH_2Cl_2$ and washed with water. The organic phase was dried ($Na_2SO_4$) and concentrated to provide the crude product which was purified by passing through 65 g of coarse $SiO_2$ column, using $CHCl_3$:MeOH:AcOH (990:9:1) as eluent to yield 0.98 g of product.

EXAMPLE 38

HEXANOIC ACID, 6,6'-[(2-PENTADECYNYLIDENE)BIS(THIO)]-BIS-

The product from preparative Example II (2.0 g) in $CH_2Cl_2$ (15 ml) was treated with 1-mercapto-5-(methoxycarbonyl)-pentane (3.9 g) under $N_2$ at room temperature. To this was added $Me_3SiCl$ (1.5 ml) and the reaction was stirred at room temperature for 1/2 hour. The solvent was removed under vacuum and the residue was purified on a $SiO_2$ column with gradient elution (hexane; 2% $Et_2O$/hexane; 5% $Et_2O$/hexane; 10% $Et_2O$/hexane) to give 3.2 g of product.

EXAMPLE 39

1-PROPYNE, 3,3'-[(2-PENTADECYNYLIDENE)-BIS(OXY)]BIS-

5.0 g of 2-pentadecynal diethyl acetal (preparative Example I), 13 ml of propargylic alcohol, and 0.2 g of p-toluene sulfonic acid were refluxed in 200 ml of benzene using a Dean-Stark trap provided with an adapter filled with Dririte. After distilling 170 ml of azeotrope, the residue was diluted with hexanes (200 ml). The organic phase was washed first with aqueous $NaHCO_3$, then with water, dried ($Na_2SO_4$) and evaporated in vacuo to provide the crude product. It was purified by column chromatography ($SiO_2$) eluting first with hexanes (1000 ml), then 3% EtOAc/hexanes. Yield: 2.4 g.

EXAMPLE 40

1-BUTANOL, 4,4'-[(TRIDECYLIDENE)BIS(OXY)]BIS-, DIBENZOATE

To a 3-neck, 100 ml reaction vessel were added: catalytic amount of p-toluenesulfonic acid monohydrate (50 mg), 8.54 g of butane-1,4-diol monobenzoate, and 5 g tridecanal, all in a total of 25 ml benzene. The reaction mixture was heated to reflux with azeotropic removal of water. After about 2 hours the reaction was cooled and the benzene solution washed with aqueous $K_2CO_3$ followed by distilled water. The organic phase was dried over $Na_2SO_4$ and evaporated to dryness in vacuo. The crude product so obtained was chromatographed on coarse silica gel (500 g) using 5% acetone in n-hexane as eluent. Fractions 14 and 15 (200 ml each) were evaporated in vacuo to provide pure dibenzoate (4.23 g) as a thick oil.

EXAMPLE 41

1-BUTANOL, 4-4'-[(TRIDECYLIDENE)BIS(OXY)]BIS-

A solution of the dibenzoate (1 g) from Example 41) in ethanol (20 ml) was treated with aqueous 10% NaOH solution. An additional 20 ml ethanol was added to obtain a homogenous solution. After stirring at room temperature for about 48 hours, the reaction was worked up as in Example 3 to provide virtually pure product as a yellowish oil. It was used as such in the next Example.

EXAMPLE 42

1-BUTANOIC ACID, 4,4'-[(TRIDECYLIDENE)BIS(OXY)]BIS-, DIMETHYL ESTER

The diol (1 g) from Example 42 was oxidized with pyridinium dichromate (7.33 g) in dry DMF (14 ml) as in Example 4A to provide crude diacid which was treated with diazomethane as in Example 4B. Chromatography of the crude dimethylester on TLC grade silica gel (30 g) using 5% acetone in n-hexane as eluent provided the pure dimethylester (0.28 g) as a yellow oil.

EXAMPLE 43

1-BUTANOIC ACID, 4,4'-[(TRIDECYLIDENE)BIS(OXY)]BIS-

A solution of the dimethylester (0.2 g) from Example 43 in ethanol was treated with 10% aqueous NaOH (2 ml) and the mixture refluxed for 4 hours. Work-up of the reaction as in Example 5 yielded the pure diacid

(0.15 g) as a crystalline solid, m.p. 33° C.

EXAMPLE 44

6-[3-(CARBOXYPROPYL)THIO]-7-EICOSYNOIC ACID

A mixture of methyl 6-bromo-7-eicosynoate (3.0 g), methyl γ-mercaptobutyrate (1.0 g) and $Cs_2CO_3$, (2.44 g) in DMF (70 ml) was stirred at room temperature for 2 hours. Dilution with water followed by extraction with $Et_2O$ afforded 4.0 g crude product which was purified on $SiO_2$ (200 g) column. Elution with 50% hexane/$CHCl_3$ gave 1.7 g pure eicosynoate.

This diester (1.42 g) was hydrolyzed with 10 ml 10% KOH and 20 ml EtOH at room temperature for 5 hours. After work-up as in Example 5, the pure diacid was obtained as a solid (1.22 g).

EXAMPLE 45

6-[5-(CARBOXYPENTYL)THIO]-7-EICOSYNOIC ACID

A mixture of methyl 6-bromo-7-eicosynoate and 1-mercapto-5-(methoxycarbonyl)-pentane (1.21 g) in 70 ml DMF was treated with $Cs_2CO_3$ (2.44 g) as in Example 45 to give 1.02 g of methyl 6-(methoxycarbonylpentylthio)-7-eicosynoate.

This diester (0.72 g) was hydrolyzed in a similar manner as in Example 45 to give the title compound as a solid (0.53 g).

EXAMPLE 46

6-[2-(CARBOXYETHYL)THIO]-7-EICOSYNOIC ACID

To a stirred slurry of 0.58 g of NaH (50%) in $Et_2O$ (12 ml) at 0° C was added dry $CF_3CH_2OH$ (0.93 ml). After the $H_2$ evolution had ceased, a solution of triphenylphosphine (1.58 g) in 12 ml of $CH_2Cl_2$ was added. After stirring for 10 min, 0.3 ml bromine was added. This mixture was then stirred at 0° C for 1 hour, followed by addition of a solution of the product from preparative Example VI (1.7 g) and methyl 3-mercaptopropionate (0.55 ml) in $CH_2Cl_2$ (2.5 ml). The mixture was stirred at room temperature for 7 hours. The reaction was quenched with water and extractive isolation with $CH_2Cl_2$ yielded 2.5 g crude product. Chromatography on 100 g $SiO_2$ using 30% hexane in $CH_2Cl_2$ as eluent gave 0.66 g of methyl 6-[(3-methoxy-1-oxopropyl)thio]-7-eicosynoate.

The above product (1.3 g) was hydrolyzed with 12.2 ml of 10% NaOH in 25 ml EtOH at room temperature for 17 hours. Work-up as in Example 5 yielded 0.91 g of the product as a yellowish solid.

EXAMPLE 47

6-[2-(TRIFLUOROACETAMIDO)ETHYLTHIO]-7-EICOSYNOIC ACID POTASSIUM SALT

The product from Example 49 (0.5 g) in 40 ml dry MeOH was treated with 0.5 g of S-ethylthiotrifluoroacetate and was stirred at room temperature for 1 hour. The MeOH was removed in vacuo to give 0.57 g of the title compound as a yellowish liquid.

EXAMPLE 48

6-(2-AMINOETHYLTHIO)-7-EICOSYNOIC ACID, POTASSIUM SALT

Methyl 6-[2-(trifluoroacetamido)ethylthio]-7-eicosynoate was prepared in a manner similar to Example 47, except that 2-(trifluoroacetamido)-ethanethiol was used instead of methyl 3-mercaptopropionate.

The above product (1.0 g) was hydrolyzed with 50 ml of 0.13 M $K_2CO_3$ in MeOH/$H_2O$ (3:1) at room temperature for 48 hours and subjected to purification on a XAD-4 (250 g) column to yield 0.56 g of product.

EXAMPLE 49

(±)-TRANS-6-(2-AMINO-3-HYDROXY-3-OXOPROPYLTHIO)-5-HYDROXY-7-EICOSYNOIC ACID, DIPOTASSIUM SALT

Hydrolysis of (±)-5-hydroxy-6-[3-hydroxy-3-oxo-2-(trifluoroacetylamino)-propylthio]-7-eicosynoic acid, dimethyl ester (0.23 g) in a manner similar to Example 49 provided 0.16 g of the title compound.

EXAMPLE 50

(±)-6-(2-AMINO-3-METHOXY-3-OXOPROPYLTHIO)-7-EICOSYNOIC ACID

(The synthesis of the title compound is described in part E of this Example.)

Part A. Cyclohexanone t-butyldimethylsilyl enolether

To a solution of dry cyclohexanone (20 g) in dry $Et_3N$ (125 ml) and dry DMF (125 ml) was added in one portion 61.2 g of t-butyldimethyl chlorosilane. The mixture was refluxed under $N_2$ for two days. After cooling to room temperature, the mixture was diluted with $Et_2O$, washed with aqueous $NaHCO_3$, then with 0.1 N HCl, then again with saturated aqueous $NaHCO_3$, and finally with water, and dried over $Na_2SO_2$. The oil, resulting from the distillation of the solvents, was purified by fractional distillation. Yield: 22 g. B.p = 70-73° C/4 mm, Hg.

Part B. t-Butyldimethylsilyl 5-formylpentanoate

A solution of 10 g of the product from part A in 150 ml dry $CH_2Cl_2$ and 15 ml t-butanol was ozonized at -78° C for 1/2 hour. The excess $O_3$ was kept in solution for 15 more minutes, then bubbled off with $N_2$. $(CH_3)_2S$ (10 ml) was added at -78° C, then the solution was allowed to warm to room temperature and kept overnight. After evaporation of the solvent, the resulting oil was purified by fractional distillation. After distilling the fraction boiling at 44-50° C/4mm Hg, the residue was used as such.

Part C. (±)-6-Hydroxy-7-eicosynoic acid t-butyldimethylsilyl ester

14.8 ml of a 3.0 molar solution of EtMgBr in $Et_2O$ was added at room temperature with stirring under $N_2$ to 10.0 gr of 1-tetradecyne in 20 ml of dry $Et_2O$. After the addition, the solution was stirred for another hour, then added to a precooled solution (dry ice-acetone bath) of the aldehyde prepared as described in part B (10.0 g) in 75 ml of dry $Et_2O$. After the addition, the thick paste formed was allowed to warn to room temperature and stirred for one hour; then 75 ml of saturated aqueous $NH_4Cl$ was added in portions. The organic layer was separated, and the aqueous layer extracted several times with $Et_2O$. The combined extracts were washed with water and dried over $Na_2SO_4$. The oil resulting from the evaporation of the solvent, was purified by fractional distillation.

The fractions distilling between 80° and 100° C at 3 mm Hg were discarded. The residue was used as such for part D.

Part D. 6-Methanesulfonyloxy-7-eicosynoic acid t-butyldimethylsilyl ester

5.0 g Of the product prepared as described in part C was dissolved in 50 ml of dry $CH_2Cl_2$ and 10 ml of dry pyridine and the solution cooled (ice bath). A solution of methanesulfonic anhydride (2.85 g) in 20 ml of dry $CH_2Cl_2$ was added dropwise. After the addition, the mixture was let warm up and stirred at room temperature for three hours, then diluted with $CH_2Cl_2$. The $CH_2Cl_2$ solution was washed twice with water, then with aqueous $NH_3$ (1:10), then again with water and dried over $Na_2SO_4$. The reaction product (5.4 g) obtained after evaporation of the solvent was used as such for part E.

Part E. (±)-6-(2-AMINO-3-METHOXY-3-OXOPROPYLTHIO)-7-EICOSYNOIC ACID

2.0 g Of the product prepared as described in part D, t-butanol (7 ml), cysteine methyl ester hydrochloride (1.46 g), and 4 ml of dry $Et_3N$ were mixed in that order under $N_2$ at room temperature. After 24 hours, 20 ml of dry $CH_2Cl_2$ was added and the mixture stirred for another 24 hours. The solvents were then evaporated, and the reaction mixture was purified by column chromatography (silica gel). The column was eluted first with $CHCl_3$ (3 liters), then with 20% $MeOH/CHCl_3$. The combined fractions containing the

compound (2.7 g) were chromatographed again on silica gel. The column was eluted successively with $CHCl_3$ (1 liter), 5% MeOH/$CHCl_3$, 10% MeOH/$CHCl_3$, and 20% MeOH/$CHCl_3$. The fractions containing the compound were combined and purified by preparative thin layer chromatography on silica gel [solvent: 10% (MeOH:$NH_3$)/$CHCl_3$ (9:1); two elutions] yield: 0.05 g.

EXAMPLE 51

(±)-6-[3-METHOXY-3-OXO-2-(TRIFLUOROACETYLAMINO)-PROPYLTHIO]-7-EICOSYNE

Part A. (±)-6-Hydroxy-7-eicosyne

The reaction conditions were the same as in part C of Example 51 except that hexanal was used instead of t-butyldimethylsilyl 5-formylpentanoate.

Part B. (±)-6-Methanesulfonyloxy-7-eicosyne

The reaction conditions were the same as in part D of Example 51.

Part C. (±)-6-[3-METHOXY-3-OXO-2-(TRIFLUOROACETYLAMINO)-PROPYLTHIO]-7-EICOSYNE

1.99 g of the product as obtained from part B, t-butanol (10 ml), 2.97 g of N-trifluoroacetyl-cysteine methyl ester and dry $Et_3N$ (2.5 ml) were reacted as described in Example 53. The reaction mixture was purified by column chromatography (silica gel) ($CHCl_3$). The fractions containing the product were combined and purified again as above using 50% $CHCl_3$/hexanes as eluent. The pure fractions were combined to provide the title compound.

EXAMPLE 52

(±)-6-[3-METHOXY-3-OXO-2-(TRIFLUOROACETYLAMINO)-PROPYLTHIO]-7-EICOSYNOIC ACID

1.97 of crude product prepared as described in part D of Example 51, t-butanol (7 ml), N-trifluoroacetyl-cysteine methyl ester (2.0 g), and $Et_3N$ (2 ml) were added at room temperature. The solution was stirred at room temperature under $N_2$ for four hours. The solvents were then evaporated under reduced pressure and the dark oily residue obtained was treated with water and extracted with $CHCl_3$. The $CHCl_3$ extract was washed with water, dried ($Na_2SO_4$) and evaporated to dryness. The reaction product (3.6 g) was purified by column chromatography (silica gel). The column was successively eluted with $CHCl_3$ and 10% MeOH/$CHCl_3$. The fractions containing the title compound were combined and purified again as above. The column was eluted first with $CHCl_3$, then with 2% MeOH/$CHCl_3$. The fractions containing the pure product were combined and evaporated in vacuo to provide the title compound.

EXAMPLE 53

(±)-6-(2-AMINO-3-HYDROXY-3-OXOPROPYLTHIO)-7-EICOSYNE, POTASSIUM SALT

0.64 g of compound from Example 52 was stirred at room temperature under $N_2$ with 60 ml of 0.13M $K_2CO_3$ in MeOH:$H_2O$ (3:1) for 16 hours. Aqueous KOH (0.35 g/ml water) was added and the reaction stirred for 20 hours at room temperature. The solvents were evaporated under high vacuum. The solid residue was treated with water (25 ml) and the pH of the solution adjusted to 5.2. The mixture was taken in $CHCl_3$ and the aqueous layer was separated. The organic layer was washed with water and dried over $Na_2SO_4$. Evaporation of the solvent gave the title compound.

EXAMPLE 54

(±)-6-(2-AMINO-3-HYDROXY-3-OXOPROPYLTHIO)-7-EICOSYNOIC ACID, DIPOTASSIUM SALT

0.4818 g of product from Example 53 was treated with 20 ml MeOH:$H_2O$ (3:1) and cooled in ice bath under $N_2$. 0.5 g of KOH in 3 ml of water was then added dropwise. The solution was allowed to warm up to room temperature and stirred for a total of four hours. Aqueous KOH (0.25 g/ml) was added and after

another hour the solvents were evaporated under reduced pressure. The residue was dissolved in water (10 ml) and applied to an XAD-4 column (145 g). The column was successively eluted with water (10 x 20 ml), 30% MeOH/$H_2O$ (6 x 20 ml), 50% MeOH/$H_2O$ (5 x 20 ml) and MeOH (11 x 20 ml). The methanolic fractions gave 0.28 g of the title compound.

EXAMPLE 55

(±)-6-[3-HYDROXY-3-OXO-2-(TRIFLUOROACETYLAMINO)-PROPYLTHIO]-7-EICOSYNOIC ACID

0.4 g of compound prepared according to Example 53 was stirred at room temperature under $N_2$ in 57 ml of 0.13M $K_2CO_3$ in MeOH:$H_2O$ (3:1) for five hours. The solvents were evaporated under high vacuum and the residue was purified using 250 g XAD-4 resin column in water. The column was eluted with water to pH=7, then with 250 ml of 30% MeOH/$H_2O$, 500 ml of 50% MeOH/$H_2O$, and 100% MeOH. The product (0.16 g) was further purified by preparative thin layer chromatography on $SiO_2$ (40% MeOH/$CHCl_3$). Yield: 0.035 g.

EXAMPLE 56

(±)-6-[3-HYDROXY-3-OXO-2-(TRIFLUOROACETYLAMINO)-PROPYLTHIO]-7-EICOSYNOIC ACID DIMETHYL ESTER

0.7 g of the product prepared according to Example 53 was treated at 0° C in $Et_2O$ with a large excess of $CH_2N_2$ in $Et_2O$. The solvent was evaporated off under $N_2$ at room temperature. The reaction mixture was purified by column chromatography ($SiO_2$) ($CHCl_3$). Yield: 0.22 g.

EXAMPLE 57

(±)-1-(TETRAHYDRO-2H-PYRAN-2-YLOXY)-6-[6-(TETRAHYDRO-2H-PYRAN-2-YLOXY)-HEXYLOXY]-7-EICOSYNE

Part A (±)-6-Hydroxy-1-(tetrahydro-2H-pyran-2-yloxy)-7-eicosyne

21 ml 2.85M EtMgBr in $Et_2O$ was added dropwise to a solution of 1-tetradecyne in 120 ml of dry $Et_2O$ at room temperature. After the addition, the brown solution was stirred at room temperature for one hour, then cooled (dry ice-acetone bath). 10.0 g 6-(tetrahydro-2H-pyran-2-yl)-hexanal in 50 ml of dry $Et_2O$ was added in one portion. The mixture was allowed to warm up to room temperature and stirred for two hours. 3.5 g $NH_4Cl$ in 30 ml water was then added. The mixture was diluted with $Et_2O$ (total volume = 750 ml), washed with water, and dried over $Na_2SO_4$. The crude product (20 g) resulting from the evaporation of the solvent was filtered through silica gel (350 g) using $CHCl_3$ as eluent. The product obtained (3.6 g) was used as such for part B.

Part B (TITLE COMPOUND)

0.9 g NaH (50% oil dispension) was washed with hexanes under $N_2$, then 4 ml of dry THF was added. 2.66 g of the product from part A was added at room temperature, and the mixture refluxed under $N_2$ for three hours. After cooling to room temperature, 3.0 g of 1-iodo-6-(tetrahydro-2H-pyran-2-yloxy)-hexane was added and the mixture refluxed overnight under $N_2$. The mixture was then cooled in ice bath and treated with water. The solution was taken in $CH_2Cl_2$ (300 ml), washed with water to pH=7 and dried over $Na_2SO_4$. The reaction product was purified by column chromatography (silica gel) (5% EtOAc/Hexanes). Yield: 1.0 g.

EXAMPLE 58

(±)-6-(6-HYDROXYHEXYLOXY)-7-EICOSYN-1-OL

1.0 g (±)-1-(tetrahydro-2H-pyran-2-yloxy-6-[6-(tetrahydro-2H-pyran-2-yloxy)-hexyloxy]-7-eicosyne (Example 58) was stirred at room temperature for seven hours with 15 ml methanol containing 50 mg of p-toluenesulfonic acid. The solution was kept overnight in the refrigerator, then treated with 3 ml MeOH:aq. $NH_3$ (8:2). After evaporation of the solvents, the resulting oil was taken up in $CH_2Cl_2$, washed with water (3 x

50 ml), and dried over $Na_2SO_4$. Yield: 0.65 g.

EXAMPLE 59

(±)-6-(5-CARBOXYPENTYLOXY)-7-EICOSYNOIC ACID DIMETHYL ESTER

The product from Example 59 was oxidized and methylated using the same procedure as described in Example 11. The reaction mixture was purified by column chromatography on silica gel (5% EtOAc/hexanes).

EXAMPLE 60

(±)-6-(5-CARBOXYPENTYLOXY)-7-EICOSYNOIC ACID

The product from Example 60 (0.2 g) was hydrolysed using the same procedure as described in Example 13. Yield: 0.195 g.

EXAMPLE 61

METHYL (-)-(5R,6S)-5-HYDROXY-6-[(2R)-2-(TRIFLUOROACETYLAMINO)-2-(METHOXYCARBONYL)-ETHYLTHIO]-7-EICOSYNOATEAND METHYL (+)-(5S,6R)-5-HYDROXY-6-[(2R)-2-(TRIFLUOROACETYLAMINO)-2-(METHOXYCARBONYL)-ETHYLTHIO]-7-EICOSYNOATE

A solution of methyl trans-5,6-epoxy-7-eicosynoate (0.3762 g) (preparative Example X) in methanol (0.1 ml) containing $Et_3N$ (0.8 ml) was treated with N-trifluoroacetyl-cysteine methylester (0.516 g). After 2 hours solvents were evaporated in vacuo and the residue distributed between water and $CH_2Cl_2$. The organic phase was then separated and the aqueous phase extracted three times with $CH_2Cl_2$. Combined $CH_2Cl_2$ extracts were washed once with water, dried ($Na_2SO_4$) and evaporated to dryness in vacuo to provide a thick yellow oil (1.0097 g). The reaction was repeated exactly as above using 1.22 g. trans-epoxide to provide another batch of the product (3.228g). The two products were combined and chromatographed on TLC grade silica gel using 20-30% ethylacetate in n-hexane as eluent:

A. Less polar isomer 0.94 g, $[\alpha]_D^{26}$ -10.9° ($CHCl_3$)

B. More polar isomer 0.66 g, $[\alpha]_D^{26}$ +22.1° ($CHCl_3$)

Both isomers were obtained as waxy solids.

EXAMPLE 62

(-)-(5R,6S)-5-HYDROXY-6-[(2R)-2-AMINO-2-CARBOXYETHYLTHIO]-7-EICOSYNOIC ACID DIPOTASSIUM SALT

The less polar dimethylester from Example 62 (0.4 g) was stirred with 90 ml 0.13M $K_2CO_3$ in MeOH:water (3:1) at room temperature under $N_2$. After 36 hours solvents were removed in vacuo (bath temp. 40° C) and the residue obtained subjected to purification on a XAD-4 (160 g) column:

Fractions: water (800 ml) - discarded
1-5 MeOH (each 400 ml) 0.2783 g
amorphous solid $[\alpha]_D^{26}$ -24.6° (MeOH).

EXAMPLE 63

(±)-(5S,6R)-5-HYDROXY-6-[(2R)-2-AMINO-2-CARBOXYETHYLTHIO]-7-EICOSYNOIC ACID DIPOTASSIUM SALT

The more polar dimethylester (0.3 g) from Example 62 was stirred with 66 ml 0.13M $K_2CO_3$ in MeOH:water (3.1) at room temperature under $N_2$. After 36 hours the reaction was worked up as in Example 63 and the product subjected to purification on a XAD-4 (120 g) column:

Fractions: water (600 ml)
1-5 MeOH (each 400 ml) 0.2264 g
amorphous solid $[\alpha]_D^{26}$ +11.9° (MeOH)

EXAMPLE 64

6-HYDROXY-6-(1-TETRADECYNYL)-1,11-UNDECANEDIOIC ACID (CRUDE)

A stirred solution of 1-tetradecyne (58.2 g) in 55 ml dry tetrahydrofuran (THF) was treated (under argon) dropwise with n-BuLi (1.6 M in n-hexane) until 85 ml had been added. A very heavy precipitation of Li-salt of tetradecyne took place causing difficulty in stirring the reaction mixture. After stirring the reaction mixture in an ice bath for about 45 minutes, 6-keto-undecanedioic acid (10.4 g) was added as a concentrated solution in dry THF (50 ml). The reaction mixture was gradually allowed to warm up to room temperature and stirred for a total of 15 hours. The pasty reaction mixture was quenched with water and extracted with n-hexane to remove excess tetradecyne. The aqueous phase was adjusted to about pH2 with aqueous oxalic acid and extracted with $CH_2Cl_2$. A crystalline solid not soluble in either phase later found to be unchanged keto-dicarboxylic acid was removed by filtration. The $CH_2Cl_2$ extract was dried ($Na_2SO_4$) and evaporated to dryness to provide a crystalline solid. Yield: 6.36 g. This product was used as such in the next Example.

EXAMPLE 65

6-HYDROXY-6-(1-TETRADECYNYL)-1,11-UNDECANEDIOIC ACID, DIMETHYLESTER

The crude diacid from Example 65 (0.6 g) was treated with excess diazomethane as in Example 4B and subjected to chromatography on six 1mm thick silica gel plates (solvent system: 20% ethylacetate in n-hexane). The less polar major band was extracted with 20% MeOH/$CHCl_3$ to provide a crystalline solid, m.p. 35° C. Yield: 0.44 g.

EXAMPLE 66

6-HYDROXY-6-(1-TETRADECYNYL)-1,11-UNDECANEDIOIC ACID (PURE)

A solution of the pure diester from Example 66 (0.22 g) in ethanol (5 ml) was treated with 10% aqueous NaOH (2.5 ml). The mixture was stirred for 36 hours at room temperature. Work-up of the reaction as in Example 5 yielded a crystalline solid, m.p. 65-66° C. Yield: 0.191 g.

EXAMPLE 67

6-(1-TETRADECYNYLIDENE)-1,11-UNDEC-5Z-ENE-DIOIC ACID DIMETHYLESTER

A solution of the tert. alcohol-dimethylester from Example 66 (1 g) in $CH_2Cl_2$ (50 ml) was treated with cooling (bath temp 0-5° C) and good stirring with $P_2O_5$ (0.8 g). After 1 hour the reaction was quenched with water. The $CH_2Cl_2$ phase was separated and the aqueous phase was extracted twice with more $CH_2Cl_2$. The combined $CH_2Cl_2$ extract was washed with aqueous $NaHCO_3$, dried over $Na_2SO_4$ and evaporated to dryness to provide the crude product (1.035 g) as a thick oil. It was purified by chromatography on coarse $SiO_2$ (30 g) using 10% ethylacetate in n-hexane as eluent ( 50 ml fractions). The desired 5Z-olefin was obtained as the less polar component. Yield: 0.7564 g. The more polar fraction was a mixture of 5Z-(major) and 5E-(minor) olefins (0.2028 g).

EXAMPLE 68

6-(1-TETRADECYNYL)-1,11-UNDEC-5Z-ENE-DIOIC ACID

A solution of the dimethylester from Example 68 (0.2 g) in ethanol (5 ml) was treated with 10% aqueous NaOH (2.5 ml). The reaction mixture was stirred for 36 hours and worked up as in Example 5 to provide the title compound as a crystalline solid, m.p. 47-48° C. Yield: 0.196 g.

EXAMPLE 69

6-(1-TETRADECYNYL)-1,11-UNDEC-5E-ENE-DIOIC ACID DIMETHYLESTER

Dehydration of 7 g. tert. alcohol-dimethylester (from Example 66) was conducted as in Example 68. The minor more polar 5E-olefin was isolated by repeated chromatography on TLC grade silica gel using 10% ethylacetate in n-hexane as eluent. The partially purified 5E-olefin (0.149 g) was further purified by preparative thin layer chromatography.

EXAMPLE 70

6-(1-TETRADECYNYL)-1,11-UNDEC-5E-ENE-DIOIC ACID

Hydrolysis of the 5E-olefin-dimethylester (0.16 g, Example 70) as in Example 69 gave the title compound as a crystalline solid. Yield: 0.15 g.

EXAMPLE 71

6-FLUORO-6-(1-TETRADECYNYL)-1,11-UNDECANEDIOIC ACID, DIMETHYLESTER

A stirred solution of the 6-hydroxy-dimethylester (1 g; Example 66) in $CH_2Cl_2$ (5 ml) was cooled (ice bath) and treated with diethylaminosulfurtrifluoride (0.7 ml; excess). After 30 minutes in the ice bath, the reaction was allowed to warm up to room temperature. The reaction mixture was stirred for a total of one-and-a-half hours followed by treatment with dilute $NaHCO_3$ solution. The $CH_2Cl_2$ layer was separated and the aqueous phase extracted once more with $CH_2Cl_2$. The combined organic extracts were washed once with water, dried ($Na_2SO_4$) and evaporated to dryness to provide a gummy product. This was chromatographed on TLC grade $SiO_2$ (50 g) using 10% ethylacetate in n-hexane as eluent (about 5 ml fractions). The desired 6-fluoro-dimethylester was isolated from the more polar fractions 47-52 as a colorless oil. Yield: 0.4856 g.

EXAMPLE 72

6-FLUORO-6-(1-TETRADECYNYL)-1,11-UNDECANEDIOIC ACID

A solution of the fluoro-diester from Example 72 (0.22 g) in ethanol was hydrolysed with 10% equeous NaOH (3 ml) as in Example 5. Work-up in the same manner provided the desired diacid as a crystalline solid, m.p. 98-100 ° C. Yield: 0.2051g.

EXAMPLE 73

6-FLUORO-6-(TETRADECYL)-1,11-UNDECANEDIOIC ACID DIMETHYLESTER

A solution of the acetylenic alcohol (0.4 g); from Example 73) in n-hexane (40 ml) was hydrogenated in the presence of 10% Pd/C (0.1 g). After 15 hours the catalyst was removed by filtration, washed with $CH_2Cl_2$ and the combined filtrates and washings were evaporated to dryness to provide a thick colorless oil. Yield: 0.4 g.

EXAMPLE 74

6-(TETRADECYL)-1,11-UNDECANEDIOIC ACID DIMETHYLESTER

A solution of the unsaturated diester (0.3 g; example 68) in n-hexane (30 ml) was hydrogenated in the presence of 10% Pd/C (0.1 g) overnight. Catalyst was removed by filtration and washed with $CH_2Cl_2$. Evaporation of combined filtrates gave the title compound as a thick colorless oil. Yield: 0.3 g.

EXAMPLE 75

6-(TETRADECYL)-1,11-UNDECANE-DIOIC ACID

A solution of the diester (0.2 g; from Example 75) in ethanol (5 ml) was treated with 10% aqueous NaOH (2.5 ml) exactly as in Example 5 to provide a crystalline solid, m.p. 48-50 ° C.

EXAMPLE 76

6-HYDROXY-6-(TETRADECYL)-1,11-UNDECANEDIOIC ACID

A solution of the saturated diester (0.23 g; Example 74) in ethanol (8 ml) was treated with 10% aqueous NaOH (2.5 ml) as in the previous experiment. The product after trituration with n-hexane provided a crystalline solid, m.p. 79-80°C. Yield: 0.1896 g.

EXAMPLE 77

6-(1-TETRADECYNYL)-1,11-UNDECANEDIOIC ACID

(The synthesis of the title compound is described in part E of this Example.)

Part A. Hexacarbonyl-dicobalt complex of 6-(1-tetradecynyl)-1,11-undec-5Z-ene-dioic acid dimethyl ester

To a solution of 6-(1-tetradecynyl)-1,11-undec-5Z-ene-dioic acid dimethylester (1.0g.; Example 68) was added (under $N_2$) dicobalt octacarbonyl (0.94 g.) in dry $CH_2Cl_2$ at room temperature. The reaction was stirred for 1 hr. followed by removal of $CH_2Cl_2$ under $N_2$ atmosphere.

Part B. Crude hexacarbonyl-dicobalt complex of title compound dimethyl ester (contains unchanged starting material)

A solution of the product from the above reaction (1.5 g.) in dry methanol (25 ml) was added to a slurry of potassium diazodicarboxylate (5.06 g.) in dry methanol (25 ml) in an atmosphere of $N_2$. The reaction mixture was cooled (ice bath) and a solution of glacial acetic acid (2.7 ml) in dry methanol (7.3 ml) was added dropwise. The mixture was stirred in the ice bath for three hours. Four additions of potassium diazodicarboxylate (as a solid) (3.3 g.) along with glacial acetic acid (1.5 ml) in dry methanol (8.5 ml) were necessary at three hour intervals. After evaporation of methanol in vacuo the residue was dissolved in $CHCl_3$, washed with aqueous $NaHCO_3$ and water and dried ($Na_2SO_4$). Evaporation of the solvent in vacuo left the crude product (1.07 g.).

PART C. Crude title compound dimethyl ester (contains unchanged starting material)

A portion of the reaction product from above (0.722 g.) was dissolved in acetone (25 ml) and the solution cooled (ice bath). Ceric ammonium nitrate (3.3g.) was added in small portions over a period of 30 minutes with good stirring. The reaction mixture was stirred for an additional 15 minutes followed by addition of n-hexane (200 ml). The organic phase was washed with water, dried ($Na_2SO_4$) and evaporated to dryness to provide the crude dimethylester of the title compound containing unchanged olefinic dimethylester (from Example 68).

Part D. Isolation of title compound dimethyl ester from unchanged starting material by epoxidation of the latter.

The above mixture was treated with m-chloroperbenzoic acid as described in preparative Example XII. The unchanged olefinic dimethylester was thus converted into its epoxide; the dimethylester of the title compound did not react. The two compounds were separated by chromatography on t.l.c. grade silica gel. The pure dimethylester of the title compound so obtained was treated as follows.

Part E TITLE COMPOUND

A portion (0.1g.) of 6-(1-tetradecynyl)-1,11-undecanedioic acid dimethylester (as obtained from Part D) was treated with 10% aqueous KOH as in Example 13 to provide the title compound. Yield: 0.09 g.

EXAMPLE 78

HEPTANOIC ACID, 6,6'-[1-PENTADECYNYLIDENE-BIS(OXY)]-BIS-, (2,2-DIMETHYL-1-OXOPROPOXYMETHYL)ESTER

To a stirred solution of heptanoic acid, 6,6'-[2-pentadecynylidene-bis(oxy)]bis- (0.5 g) in dry DMF (3 ml) was added dry $Et_3N$ (0.77 ml). The solution was cooled in an ice bath and a solution of chloromethyl pivalate (0.34 ml) was added. The solution was allowed to warm to room temperature and stirred in an argon atmosphere for about 15 hrs. The solvent was evaporated in vacuo, the residue treated with water (20 ml) and extracted several times with ethyl acetate. The combined extracts were washed with water, dried ($Na_2SO_4$) and evaporated to dryness to provide a gummy product. The impure product was filtered through a column of $SiO_2$ (30 g) using $CHCl_3$ as eluent. Yield: 0.7 g.

PREPARATIVE EXAMPLE I

2-PENTADECYNAL DIETHYLACETAL

1-Tetradecyne (100 g), $(EtO)_3CH$ (200 ml) and $ZnI_2$ (15 g) were heated together (bath temperature 170-175°) with distillative removal of ethanol (about 90 minutes). The reaction mixture was evaporated in vacuo (bath temperature 80°) to remove excess $(EtO)_3CH$. The residue was distributed between $CH_2Cl_2$ and water. The $CH_2Cl_2$ phase was separated, washed with aqueous $NaHCO_3$, dried ($Na_2SO_4$) and evaporated in vacuo to provide a light brown oil. Yield: 135.4 g. This product was shown to be virtually pure by TLC and PMR and it was used as such in subsequent reactions.

PREPARATIVE EXAMPLE II

2-PENTADECYNAL

A mixture of 2-pentadecynal diethyl acetal (4g) and 10% aqueous $H_2SO_4$ (60 ml) was refluxed with efficient stirring for one hour. An additional 40 ml dilute $H_2SO_4$ was added at this stage and the mixture heated for another two hours. After cooling, the reaction mixture was extracted with $CH_2Cl_2$, the extract dried ($Na_2SO_4$) and evaporated in vacuo to provide a yellow oil. Yield: 2.9 g.

PREPARATIVE EXAMPLE III

5-FORMYL-4E-ENE-2-PENTYNAL DIETHYL ACETAL

A stirred solution of diformyl acetylene monodiethylacetal (2.28 g) was treated in small portions with $Ph_3P=CH.CHO$ (4.47 g). After stirring for 15 hours the solvent was evaporated in vacuo and the dark residue subjected to chromatography on coarse $SiO_2$ (100 g) using 5% acetone/n-hexane as eluent. Fractions (100 ml) were collected and the title compound was obtained pure from fraction no. 6. Yield: 2.01 g.

PREPARATIVE EXAMPLE IV

4E,6Z,9Z-PENTADECATRIENE-2-YNAL DIETHYL ACETAL

A solution of 3-(Z)-nonene-triphenylphosphonium salt in 20-23 ml of dry THF was treated with 1.6M BuLi (6.79 ml) and stirred at room temperature for 1 hour. The aldehyde (1.68 g, Example III) in 3 ml THF was added and allowed to stir for another 3 hours. The reaction mixture was diluted with EtOAc and washed with dilute $NaHSO_3$ solution and brine, and dried ($Na_2SO_4$).Evaporation of the solvent followed by chromatography on $SiO_2$ (eluent 10% $Et_2O$ in n-hexane) gave the pure product (1.17 g) as a yellow oil.

PREPARATIVE EXAMPLE V

(±)-6-HYDROXY-7-EICOSYNOIC ACID METHYL ESTER

A cooled solution (ice bath) of 6-oxo-7-eicosynoic acid methyl ester (prepared by coupling 1-pentadecyne zinc chloride and methyl 6-chloro-6-oxo-hexanoate under standard conditions, e.g. R.G. Jones, J.A.C.S., 69, 2353, 1947) (29.86 g) in methanol (475 ml) and water (10 ml) was treated with $NaBH_4$ (1.27 g) in small portions. After 30 minutes the reaction was diluted with water (50 ml), methanol evaporated in vacuo and the residue distributed between water (50 ml) and $CH_2Cl_2$ (100 ml). $CH_2Cl_2$ phase was extracted twice with $CH_2Cl_2$. The combined $CH_2Cl_2$ extracts were dried ($Na_2SO_4$) and evaporated to dryness to

provide the almost pure alcohol as a thick oil (30.23 g). It was further purified on coarse silica gel (300 g) using 5% ethyl acetate/n-hexane as eluent. Fractions 7-18 (250 ml each) gave the pure alcohol. Yield: 21.79 g.

PREPARATIVE EXAMPLE VI

(+)-6(R)-HYDROXY-7-EICOSYNOIC ACID METHYL ESTER

A solution of (+)-α-pinene (1.04 ml; $[\alpha]_D^{26}$ +47.7°) and 9-borabicyclononane (12 ml of 0.5M THF solution; 0.006 mol) was refluxed for two-and-a-half hours and cooled to room temperature. With ice bath cooling 6-oxo-7-eicosynoic acid methyl ester (1 g; 0.003 mol) was now added. The mixture was now stirred under argon at room temperature for 3 days. Acetaldehyde (21 ml) was injected into the solution which was then stirred for 15 minutes. The THF and (+)-α-pinene were removed in vacuo at 40° (bath temperature). The remaining yellow product was dissolved in diethylether (5 ml), and ethanolamine (0.38 g; 0.006 mol) was added. After stirring at ice bath temperature for 15 minutes, the white precipitate formed was removed by filtration and washed with cold diethylether. The combined filtrate was washed with saturated NaCl, dried ($Na_2SO_4$) and evaporated to dryness to provide a thick oil. It was chromatographed on TLC grade $SiO_2$ (100 g) using 10% ethyl acetate in n-hexane as eluent. Fractions 103-135 (about 5 ml each) gave pure 6R-alcohol. Yield: 0.6268 g.

Note: The 6(S)-alcohol of opposite configuration was obtained by substituting (-)-α-pinene for (+)-α-pinene in the above reaction.

PREPARATIVE EXAMPLE VII

2-PENTADECYNOL

A stirred solution of the aldehyde from preparative Example II (16.56 g) in methanol (160 ml) and water (16 ml) was cooled (ice bath) and treated with $NaBH_4$ (1.25 g) in small portions. After about 10 minutes a crystalline precipitate separated. Stirring was continued for 30 minutes. The solvents were evaporated in vacuo and the residue was distributed between $CH_2Cl_2$ (about 100 ml) and water (about 50 ml). The $CH_2Cl_2$ phase was separated and the aqueous phase extracted twice with $CH_2Cl_2$. The combined $CH_2Cl_2$ extracts were washed once with water, dried ($Na_2SO_4$) and evaporated to dryness to provide a crystalline solid, m.p. 36-37° C. Yield: 17.8 g.

PREPARATIVE EXAMPLE VIII

1-BROMO-2-PENTADECYNE

A stirred solution of the alcohol (17 g; preparative example VII) in $CH_2Cl_2$ (500 ml) was treated with $CBr_4$ (30.19 g). After all $CBr_4$ had dissolved, the solution was cooled (ice bath) and treated with $Ph_3P$ (25.87 g). The reaction was worked up after 1 hour as follows: $CH_2Cl_2$ was evaporated in vacuo and the gummy residue treated with n-hexane. A precipitate of $Ph_3P=O$ so obtained was removed by filtration and washed with n-hexane. The combined filtrate and washings were evaporated to dryness in vacuo and passed through a column of coarse $SiO_2$ (200 g) using n-hexane (about 2 liters) as eluent. Evaporation of the n-hexane eluate in vacuo (temp. 60-90°) provided the bromide as a colorless oil. Yield: 19.9 g.

PREPARATIVE EXAMPLE IX

2-PENTADECYNE-1-TETRAMETHYLENE SULFONIUM BROMIDE

To a solution of the bromide (18.9 g.; preparative Example VIII) in 150 ml methanol:water (9:1) was added tetrahydrothiophene (7.56 g). The reaction mixture was efficiently stirred for 2 days. After washing once with n-hexane, the MeOH:water phase was evaporated to dryness and the residue dissolved in $CH_2Cl_2$. The methylene chloride solution was concentrated to about 10 ml and treated with n-hexane until separation of colorless crystals took place. The crystals were collected by filtration. Yield: 13.6 g, m.p. 76-79° C.

PREPARATIVE EXAMPLE X

TRANS-5,6-EPOXY-7-EICOSYNOIC ACID METHYL ESTER AND CIS-5,6-EPOXY-7-EICOSYNOIC ACID METHYL ESTER

A stirred and cooled solution (bath temperature -25° ) of the tetramethylene sulfonium bromide (3 g; preparative Example IX) methyl 4-formylbutyrate (1.04 g) containing benzyltriethylammonium chloride (0.054 g) in $CH_2Cl_2$ (15 ml) was treated with 10M NaOH solution (8.06 ml) in one portion. The mixture was efficiently stirred for 1 min. and then rapidly cooled to -70° (bath temp.). The $CH_2Cl_2$ layer was decanted out with a pipette. The frozen aqueous phase was washed 3-4 times with $CH_2Cl_2$, the combined $CH_2Cl_2$ extract and washings were washed with water, dried ($Na_2SO_4$) and evaporated to dryness to provide a turbid oil. The products from four such reactions were combined to yield 14.2 g total crude product which was chromatographed on TLC grade $SiO_2$. The column was eluted with 50% n-hexane/$CHCl_3$ (containing 2 ml triethylamine per liter) and about 6 ml fractions were collected:

Fractions 102-132 Pure trans-epoxide
Fractions 191-205 Pure cis-epoxide

PREPARATIVE EXAMPLE XI

METHYL (±)-6-BROMO-7-EICOSYNOATE

This compound was prepared by reaction of (±)-6-hydroxy-7-eicosynoic acid methylester (preparative Example V) with $CBr_4/Ph_3P$ reagent in exactly the same manner as described in Preparative Example VIII.

By use of the corresponding 6-R and 6-S alcohols (preparative Example VI) the optically active (+)- and (-)-6-bromo-7-eicosynoates were also obtained.

PREPARATIVE EXAMPLE XII

6-(1-TETRADECYNYL)-5,6-EPOXY-1,11-UNDECANEDIOIC ACID DIMETHYL ESTER

0.19 g. of the product from Example 70 was cooled in ice bath and meta-chloroperbenzoic acid (0.07 g) was added. Additional 0.1 g peracid was added in two portions after two and three hours. Two hours later, the reaction mixture was diluted with $CH_2Cl_2$ (180 ml), washed with aqueous $NaHSO_3$, aqueous $Na_2CO_3$ (3 x 50 ml), then with water and dried over $Na_2SO_4$. The reaction mixture was purified by column chromatography on silica gel ($CHCl_3$). The fractions containing the title compound were further purified by preparative thin layer chromatography on silica gel (20% EtOAc/hexanes). Yield: 0.139 g.

PREPARATIVE EXAMPLE XIII

(±)-DIPOTASSIUM[6-(1-TETRADECYNYL)]-5,6-EPOXY-1,11-UNDECANEDIOATE

0.37 g. 6-(1-tetradecynyl)-5,6-epoxy-1,11-undecanedioic acid dimethyl ester was stirred at room temperature with 5 ml of absolute EtOH and 1.84 ml of 10% aqueous KOH for 24 hours. The solvents were then evaporated and the mixture was desalted on a XAD-4 resin (35 g) column. The column was eluted first with water to pH = 7, then with 50% $H_2O$/MeOH to provide in methanolic fractions the title compound.

## Claims

**1.** A compound having the structural formula A

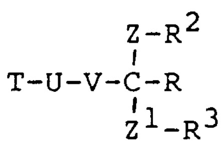

A

wherein T is straight or branched chain alkyl having from 7-15 carbon atoms which may optionally contain from 1-3 non-cumulative double or triple bonds;

U is $-CH_2CH_2-$, $-CH=CH-$ or $-C\equiv C-$;

V is straight or branched chain alkylene having from 1 to 4 carbon atoms or is a direct bond;

Z and $Z^1$ are both W-X or both Y or one is W-X and the other is Y, where W is O or $S(O)_m$, [wherein m is O, 1 or 2];

X is a straight or branched chain divalent hydrocarbon group having from 2 to 12 carbon atoms which may optionally contain from 1 to 3 non-cumulative double or triple bonds and which may be optionally substituted with the group $-NHR^a$ [wherein $R^a$ is hydrogen, alkyl having from 1 to 6 carbon atoms, $COCF_3$, $CO(CH_2)_2CH(NH_2)CO_2H$, or $SO_2R^b$ (wherein $R^b$ is alkyl having from 1 to 6 carbon atoms or $CF_3$)];

$R^2$ and $R^3$ may be the same or different and are hydrogen, $CH_2OR^c$ [wherein $R^c$ is hydrogen, carboxylic acyl having from 1 to 6 carbon atoms, tetrahydro-2H-pyran-2-yl or $COCH_2CH_2CO_2H$], CHO, 2-tetrazolyl, $COR^d$ [wherein $R^d$ is hydroxy, alkoxy having from 1 to 6 carbon atoms, $OCH_2OC(O)C(CH_3)_3$, $NHR^e$ (wherein $R^e$ is hydrogen, alkyl having from 1 to 6 carbon atoms or $CH_2CO_2H$)] or $SO_3H$, with the proviso that at least one of $R^2$ and $R^3$ is 2-tetrazolyl or carboxyl;

Y is straight or branched chain alkylene having from 1 to 12 carbon atoms which may optionally contain from 1 to 3 non-cumulative double or triple bonds;

with the following provisos:
(A) where R is hydrogen, U is $-C\equiv C-$, V is a direct bond, $-Z-R^2$ is $-S-(CH_2)_{2-3}$ COOH and $-Z^1-R^3$ is $-S-(CH_2)_{2-3}COOH$, T is other than $C_{8-13}$ alkyl;
(B) said compound of formula A is not isodecane-1, 1-bis(mercaptopropionic acid).

**2.** A compound as claimed in Claim 1 having the structural formula

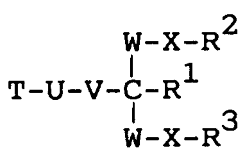

I

wherein T, U, V, W, $R^2$ and $R^3$ are as defined in Claim 1, each X is a straight or branched chain alkylene having from 2 to 12 carbon atoms which may optionally contain from 1 to 3 non-cumulative double or triple bonds and which may be optionally substituted with the group $-NHR^a$ (wherein $R^a$ is as defined in Claim 1);

and $R^1$ is hydrogen or a straight or branched chain alkyl having from 1-6 carbon atoms;
and the pharmaceutically acceptable salts thereof.

**3.** A compound as claimed in claim 2 wherein:
T is straight chain alkyl having from 7-15 carbon atoms;
U is $-C\equiv C-$;
V is a direct bond;
each W is O;
each X is a straight or branched chain alkylene having from 2 to 8 carbon atoms;
$R^1$ is hydrogen; and
$R^2$ and $R^3$ are COOH;
and their pharmaceutically acceptable salts with bases.

**4.** A compound as claimed in claim 1 having the structural formula

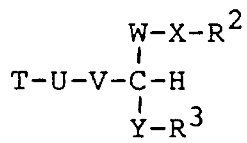

II

wherein T, U, V, W, X, Y, $R^2$ and $R^3$ are defined in claim 1;
and the pharmaceutically acceptable salts thereof.

**5.** A compound as claimed in claim 4 wherein:
T is straight chain alkyl having from 7-15 carbon atoms;
U is -C≡C-;
V is a direct bond;
W is O or S;
X and Y are straight or branched chain alkylene having from 2 to 6 carbon atoms; and
$R^2$ and $R^3$ are COOH;
and the pharmaceutically acceptable salts thereof with bases.

**6.** A compound as claimed in claim 1 having the structural formula

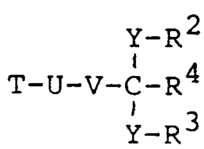

III

wherein T, U, V, Y, $R^2$ and $R^3$ are as defined in claim 1;

and $R^4$ is hydrogen or hydroxyl or can be combined with one group Y and with the mutually bonded carbon atom to form a C-Y double bond or a cyclopropane ring; and the pharmaceutically acceptable salts thereof.

**7.** A compound as claimed in claim 6 wherein:
T is straight chain alkyl having from 7-15 carbon atoms;
U is -C≡C-;
V is a direct bond;
each Y is a straight or branched chain alkylene having from 2 to 6 carbon atoms;
$R^2$ and $R^3$ are COOH; and
$R^4$ is hydrogen or hydroxyl or is combined with one group Y and with the mutually bonded carbon atom to form a double bond;
and the pharmaceutically acceptable salts thereof with bases.

**8.** A compound as claimed in any of claims 2 to 7 wherein the substituents T-U-V- taken together form the 1-tetradecynyl group, i.e. n-$C_{12}H_{25}C$≡C-, and/or wherein the substituents $R^2$ and $R^3$ may be the same or different and are $COR^d$ wherein $R^d$ is as defined in claim 1.

**9.** A compound as claimed in claim 1 having the following name:

butanoic acid, 4,4'-[(2-pentadecynylidene)bis(oxy)]bis-;

hexanoic acid, 6,6'-[(2-pentadecynylidene)bis(oxy)]bis-;

(±)-pentanoic acid, 4,4'-[(2-pentadecynylidene)bis(oxy)] bis-;

(±)-heptanoic acid, 6,6'-[(2-pentadecynylidene)bis(oxy)] bis-;

(±)-6-(4-hydroxybutoxy)-5-oxaeicos-7-ynoic acid;

hexanoic acid, 6,6'-[(2-pentadecynylidene)bis(thio)]bis-;

butanoic acid, 4,4'-[(tridecylidene)bis(oxy)]bis-;

butanoic acid, 4,4'-[(pentadecylidene)bis(oxy)]bis-;

(±)-hexanoic acid, 4,4'-[(2-pentadecynylidene)bis(oxy)] bis-;

(±)-6-(carboxyethylthio)-7-eicosynoic acid;

(±)-6-(carboxypropylthio)-7-eicosynoic acid;

(+) and (-)-6-(carboxypentylthio)-7-eicosynoic acid;

(±)-6-(carboxypentyloxy)-7-eicosynoic acid;

(±)-6-[2-(N-trifluoroacetamido)-ethylthio]-7-eicosynoic acid;

(±)-6-(2-amino-3-hydroxy-3-oxopropylthio)-7-eicosynoic acid, dipotassium salt;

(±)-6-(3-hydroxy-3-oxo-2-trifluoroacetylamino-propylthio) -7-eicosynoic acid;

6-hydroxy-6-(1-tetradecynyl)-1,11-undecanedioic acid;

6-(1-tetradecynyl)-1,11-undecanedioic acid;

6-(1-tetradecynyl)-1,11-undec-5(E)- and -(Z)ene-dioic acids;

(±)-pentanoic acid, 4,4'-[(2-pentadecynylidene)bis(thio)] bis-;

6-hydroxy-6-(tetradecyl)-1,11-undecanedioic acid;

2-butynoic acid, 4,4'-[(2-pentadecynylidene)bis(oxy)]bis-;

methanesulfonamide, N,N'-[2-pentadecynylidene-bis(oxy-5,1-hexanediyl)]bis-; or

hexanoic acid, 5-[1-(5-amino-1-methyl-5-oxopentyloxy)-2-pentadecynyloxy].

**10.** Process for the preparation of a compound or salt claimed in claim 1, in which process the symbols T, U, V, W, X, Y, $R^1$, $R^2$, $R^3$ and $R^4$, unless otherwise stated, are as defined in claim 1, with the proviso that amino, hydroxy and carboxy groups can be protected, which comprises

A) for the preparation of a compound or salt claimed in claim 2, reacting a compound of formula X or XA with a compound of formula XI

T-U-V-$CR^1(OR'')_2$ X

T-U-V-$COR^1$ XA

$HWXR^2$ XI

wherein R'' is an etherifying group;

B) for the preparation of a compound a salt claimed in claim 2 wherein one symbol W is sulfur, reacting a compound of formula I defined in claim 2 where each W is oxygen with one equivalent of a compound of formula XI defined above wherein W is sulfur with displacement of the W-X-$R^2$ or the W-X-$R^3$ substituent;

C) for the preparation of a compound or salt claimed in claim 4 wherein W is oxygen, reacting a compound of formula XII,

T-U-V-CH(OH)Y$R^3$ XII,

with a base in an anhydrous solvent medium and then with a compound of the formula Hal-X-$R^2$, wherein Hal is a halogen atom;

D) for the preparation of a compound or salt claimed in claim 4, reacting a hydrogen sulfonate ester of a compound of formula XII defined above, or the corresponding bromo or activated phosphorus derivative, with a compound of formula XIV or XV

HS-X-$R^2$ XIV

HO-X-$R^2$ XV;

E) for the preparation of a compound or salt claimed in claim 6, reacting an anion derived from a compound having formula XVII

T-U-V-H XVII

with a compound of formula XIX

$R^2$-Y-CO-Y-$R^3$ XIX,

to produce a compound of formula III defined in claim 6 wherein $R^4$ is a hydroxyl group;
whereafter the resulting compound of the formula III can if desired be reacted with diethylaminosulfur trifluoride to produce the corresponding compound wherein $R^4$ is fluorine,
or can be dehydrated to produce a compound wherein $R^4$ and one group Y are combined to form a double bond;
whereafter this double bond compound can be reduced to produce a compound of the formula III wherein $R^4$ is hydrogen, or can be reacted with methylene carbene to produce a corresponding compound having formula III wherein $R^4$ is combined with one group Y and with the mutually bonded carbon atom to form a cyclopropane ring;

whereafter the resulting compound of the formula A wherein W is sulfur can if desired be oxidized to the corresponding sulfoxide or sulfone;
and/or a compound wherein $R^2$ or $R^3$ is $CH_2OH$ can be oxidized to a compound wherein $R^2$ or $R^3$ is $CO_2OH$ or can be acylated to a compound wherein $R^2$ or $R^3$ is $CH_2OCOCH_3$; and/or any protecting groups are removed;
and the resulting compound of the formula A is isolated as such or as a pharmaceutically acceptable salt thereof.

**11.** A pharmaceutical composition which comprises a

compound having the structural formula A

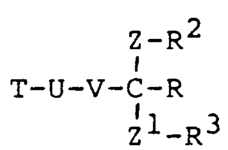

A

wherein T is straight or branched chain alkyl having from 7-15 carbon atoms which may optionally contain from 1-3 non-cumulative double or triple bonds;

U is $-CH_2CH_2-$, -CH = CH- or -C≡C-;

V is straight or branched chain alkylene having from 1 to 4 carbon atoms or is a direct bond;

Z and $Z^1$ are both W-X or both Y or one is W-X and the other is Y, where W is O or $S(O)_m$, [wherein m is O, 1 or 2];

X is a straight or branched chain divalent hydrocarbon group having from 2 to 12 carbon atoms which may optionally contain from 1 to 3 non-cumulative double or triple bonds and which may be optionally substituted with the group $-NHR^a$ [wherein $R^a$ is hydrogen, alkyl having from 1 to 6 carbon atoms, $COCF_3$, $CO(CH_2)_2CH(NH_2)CO_2H$, or $SO_2R^b$ (wherein $R^b$ is alkyl having from 1 to 6 carbon atoms or $CF_3$)];

$R^2$ and $R^3$ may be the same or different and are hydrogen, $CH_2OR^c$ [wherein $R^c$ is hydrogen, carboxylic acyl having from 1 to 6 carbon atoms, tetrahydro-2H-pyran-2-yl or $COCH_2CH_2CO_2H$], CHO, 2-tetrazolyl, $COR^d$ [wherein $R^d$ is hydroxy, alkoxy having from 1 to 6 carbon atoms, $OCH_2OC(O)C(CH_3)_3$, $NHR^e$ (wherein $R^e$ is hydrogen, alkyl having from 1 to 6 carbon atoms or $CH_2CO_2H$)] or $SO_3H$, with the proviso that at least one of $R^2$ and $R^3$ is 2-tetrazolyl or carboxyl;

Y is straight or branched chain alkylene having from 1 to 12 carbon atoms which may optionally contain from 1 to 3 non-cumulative double or triple bonds; with the proviso that, when only one group Y is present it may optionally be substituted with the group $OR^c$, where $R^c$ is as defined above;

and
R is hydrogen or, where Z and $Z^1$ are both W-X, R can also be straight or branched chain alkyl having from 1to 6 carbon atoms, or, where Z and $Z^1$ are both Y, R can also be hydroxyl or fluorine or can be combined with Z and with the mutually bonded carbon atom to form a C-Z double bond or a cyclopropane ring;

or a pharmaceutically acceptable salt thereof, in association with a pharmaceutically acceptable carrier, with the proviso that where R is hydrogen, U is -C≡C-, V is a direct bond, $-Z-R^2$ is $-S-(CH_2)_{2-3}$ COOH and $-Z^1-R^3$ is $-S-(CH_2)_{2-3}COOH$, T is other than $C_{8-13}$ alkyl.

**12.** A composition as claimed in Claim 11 in the form of dosage units.

**13.** A compound having the structural formula A

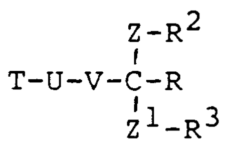

A

wherein T is straight or branched chain alkyl having from 7-15 carbon atoms which may optionally contain from 1-3 non-cumulative double or triple bonds;

U is $-CH_2CH_2-$, -CH = CH- or -C≡C-;

V is straight or branched chain alkylene having from 1 to 4 carbon atoms or is a direct bond;

Z and $Z^1$ are both W-X or both Y or one is W-X and the other is Y, where W is O or $S(O)_m$, [wherein m is O, 1 or 2];

X is a straight or branched chain divalent hydrocarbon group having from 2 to 12 carbon atoms which may optionally contain from 1 to 3 non-cumulative double or triple bonds and which may be optionally substituted with the group $-NHR^a$ [wherein $R^a$ is hydrogen, alkyl having from 1 to 6 carbon atoms, $COCF_3$, $CO(CH_2)_2CH(NH_2)CO_2H$, or $SO_2R^b$ (wherein $R^b$ is alkyl having from 1 to 6 carbon atoms or $CF_3$)];

$R^2$ and $R^3$ may be the same or different and are hydrogen, $CH_2OR^c$ [wherein $R^c$ is hydrogen, carboxylic acyl having from 1 to 6 carbon atoms, tetrahydro-2H-pyran-2-yl or $COCH_2CH_2CO_2H$], CHO, 2-tetrazolyl, $COR^d$ [wherein $R^d$ is hydroxy, alkoxy having from 1 to 6 carbon atoms, $OCH_2OC(O)C$-$(CH_3)_3$, $NHR^e$ (wherein $R^e$ is hydrogen, alkyl having from 1 to 6 carbon atoms or $CH_2CO_2H$)] or $SO_3H$, with the proviso that at least one of $R^2$ and $R^3$ is 2-tetrazolyl or carboxyl;

Y is straight or branched chain alkylene having from 1 to 12 carbon atoms which may optionally contain from 1 to 3 non-cumulative double or triple bonds; with the proviso that, when only one group Y is present it may optionally be substituted with the group $OR^c$, where $R^c$ is as defined above;

and
R is hydrogen or, where Z and $Z^1$ are both W-X, R can also be straight or branched chain alkyl having from 1to 6 carbon atoms, or, where Z and $Z^1$ are both Y, R can also be hydroxyl or fluorine or can be combined with Z and with the mutually bonded carbon atom to form a C-Z double bond or a cyclopropane ring;

or a pharmaceutically acceptable salt thereof for use in treating allergic reactions or psoriasis or inflammation in a mammal or for reducing the severity of myocardial infarction resulting from heart attack in a mammal, with the proviso that where said use comprises the treatment of allergic reactions, where R is hydrogen, U is -C≡C-, V is a direct bond, -Z-$R^2$ is -S-$(CH_2)_{2-3}$ COOH and -$Z^1$-$R^3$ is -S-$(CH_2)_{2-3}$COOH, T is other than $C_{8-13}$ alkyl.

**14.** A compound selected from the following:

1-butanol, 4,4'-[(2-nonynylidene)bis(oxy)]bis-,dibenzoate;
1-butanol, 4,4'-[(2-nonynylidene)bis(oxy)]bis-;
butanoic acid, 4,4'-[(2-nonynylidene)bis(oxy)]bis;
L-cysteine, S[R or S]-[1-(4-methoxy-4-oxobutoxy)-2-nonynyl]-N-(trifluoroacetyl)-, methylester - (less polar isomer);
and
L-cysteine, S[R or S]-[1-(4-methoxy-4-oxobutoxy)-2-nonynyl]-N-(trifluoroacetyl)-, methylester - (more polar isomer).

## Revendications

**1.** Composé ayant la formule de structure A

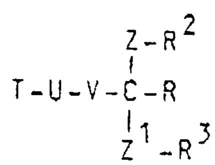

A

où T est un alkyle à chaîne droite ou ramifiée ayant 7-15 atomes de carbone qui peut facultativement contenir de 1 à 3 doubles ou triples liaisons non cumulatives ;

U est -$CH_2CH_2$-, -CH = CH- ou -C≡C- ;

V est un alkylène à chaîne droite ou ramifiée ayant 1 à 4 atomes de carbone ou bien est une liaison directe ;

Z et $Z^1$ sont tous deux W-X ou bien tous deux Y ou bien l'un est W-X et l'autre est Y, où W est O ou $S(O)_m$ [où m est 0, 1 ou 2];

X est un groupe hydrocarbure divalant à chaîne droite ou ramifiée ayant 2 à 12 atomes de carbone qui peut facultativement contenir 1 à 3 doubles ou triples liaisons non cumulatives et qui peut facultativement être substitué par le groupe -$NHR^a$ [où $R^a$ est hydrogène, alkyle ayant 1 à 6 atomes de carbone, $COCF_3$, $CO(CH_2)_2CH(NH_2)CO_2H$ ou $SO_2R^b$ (où $R^b$ est alkyle ayant 1 à 6 atomes de carbone ou bien $CF_3$)] ;

$R^2$ et $R^3$ peuvent être identiques ou différents et sont hydrogène, $CH_2OR^c$ [où $R^c$ est hydrogène,

acyle carboxylique ayant 1 à 6 atomes de carbone: tétrahydro-2H-pyran-2-yle ou $COCH_2CH_2CO_2H$], CHO, 2-tétrazolyle,$COR^d$ [où $R^d$ est hydroxy alcoxy ayant 1 à 6 atomes de carbone, $OCH_2OC(O)C-(CH_3)_3$, $NHR^e$ (où $R^e$ est hydrogène, alkyle ayant 1 à 6 atomes de carbone ou $CH_2CO_2H$)] ou $SO_3H$, à condition qu'au moins l'un de $R^2$ et $R^3$ soit 2-tétrazolyle ou carboxyle ;

Y est alkylène à chaîne droite ou ramifiée ayant 1 à 12 atomes de carbone, qui peut facultativement contenir 1 à 3 doubles ou triples liaisons non cumulatives ; aux conditions que, lorsque seul un groupe Y est présent, il puisse facultativement être substitué par le groupe $OR^c$, où $R^c$ est tel que défini ci-dessus ; et

R est hydrogène ou, quand Z et $Z^1$ sont tous deux W-X, R peut également être alkyle à chaîne droite ou ramifiée ayant 1 à 6 atomes de carbone, ou bien quand Z et $Z^1$ sont tous deux Y, R peut également être hydroxyle ou fluor ou bien peut être combiné à Z et à l'atome de carbone mutuellement lié pour former une double liaison C-Z ou un noyau de cyclopropane ;

et les sels acceptables en pharmacie ;

aux conditions qui suivent :

(A) quand R est hydrogène, U est -C≡C-, V est une liaison directe, $-Z-R^2$ est $-S-(CH_2)_{2-3}COOH$ et $-Z^1-R^3$ est $-S-(CH_2)_{2-3}COOH$, T soit autre que alkyle $C_{8-13}$,

(B) ledit composé de formule A n'est pas isodécane-1, 1-bis(acide mercaptopropionique).

**2.** Composé selon la revendication 1 ayant la formule de structure

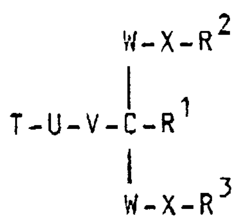

I

où T, U, V, W, $R^2$ et $R^3$ sont tels que définis à la revendication 1 chaque X est un alkylène à chaîne droite ou ramifiée ayant 2 à 12 atomes de carbone qui peut facultativement contenir 1 à 3 doubles ou triples liaisons non cumulatives et qui peut facultativement être substitué par le groupe $-NHR^a$ (où $R^a$ est tel que défini à la revendication 1) ;

et $R^1$ est hydrogène ou un alkyle à chaîne droite ou ramifiée ayant 1-6 atomes de carbone;

et leurs sels acceptables en pharmacie.

**3.** Composé selon la revendication 2 où :

T est alkyle à chaîne droite ayant 7-15 atomes de carbone ;

U est -C≡C- ;

V est une liaison directe ;

chaque W est O ;

chaque X est un alkylène à chaîne droite ou ramifiée ayant 2 à 8 atomes de carbone ;

$R^1$ est hydrogène ; et

$R^2$ et $R^3$ sont COOH ;

et leurs sels acceptables en pharmacie avec des bases.

**4.** Composé selon la revendication 1 ayant la formule de structure

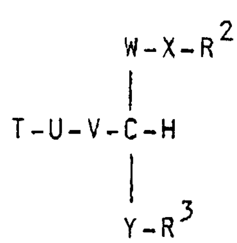

II

où T, U, V W, X, Y, $R^2$ et $R^3$ sont tels que définis à la revendication 1 ;
et leurs sels acceptables en pharmacie.

**5.** Composé selon la revendication 4 où :
T est alkyle à chaîne droite ayant 7-15 atomes de carbone ;
U est -C≡C- ;
V est une liaison directe ;
W est O ou S ;
X et Y sont alkylène à chaîne droite ou ramifiée ayant 2 à 6 atomes de carbone ; et
$R^2$ et $R^3$ sont COOH ;
et leurs sels acceptables en pharmacie avec des bases.

**6.** Composé selon la revendication 1 ayant la formule de structure

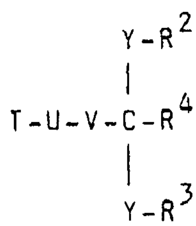

III

où T, U, V, Y, $R^2$ et $R^3$ sont tels que définis à la revendication 1 ;
et $R^4$ est hydrogène ou hydroxyle ou bien peut être combiné à un groupe Y et avec l'atome de carbone mutuellement lié pour former une double liaison C-Y ou un noyau de cyclopropane ;
et les sels acceptables en pharmacie.

**7.** Composé selon la revendication 6 où ;
T est alkyle à chaîne droite ayant 7-15 atomes de carbone ;
U est -C≡C- ;
V est une liaison directe ;
chaque Y est un alkylène à chaîne droite ou ramifiée ayant 2 à 6 atomes de carbone ;
$R^2$ et $R^3$ sont COOH ; et
$R^4$ est hydrogène ou hydroxyle ou est combiné à un groupe Y et à l'atome de carbone mutuellement lié pour former une double liaison ;
et leurs sels acceptables en pharmacie avec des bases.

**8.** Composé selon l'une quelconque des revendications 2 à 7 où les substituants T-U-V-, pris ensemble, forment le groupe 1-tétradécynyle, i.e. n-$C_{12}$,$H_{25}C=C$-, et/ou les substituants $R^2$ et $R^3$ peuvent être identiques ou différents et sont $COR^d$ où $R^d$ est tel que défini à la revendication 1.

**9.** Composé selon la revendication 1 ayant le nom suivant :
acide butanoïque, 4,4'-[(2-pentadécynylidène)bis(oxy)]bis-;
acide hexanoïque, 6,6'-[(2-pentadécynylidène)bis(oxy)]bis-;
acide (±)-pentanoïque, 4,4'-[(2-pentadécynylidène)bis(oxy)] bis-;
acide (±)-heptanoïque, 6,6'-[(2-pentadécynylidène)bis(oxy)] bis-;
acide (±)-6-(4-hydroxybutoxy)-5-oxaéicos-7-ynoïque;
acide hexanoïque, 6,6'-[(2-pentadécynylidène)bis(thio)]bis-;
acide butanoïque, 4,4'-[(tridécylidène)bis(oxy)]bis-;
acide butanoïque, 4,4'-[(pentadécylidène)bis(oxy)]bis-;
acide (±)-hexanoïque, 4,4'-[(2-pentadécynylidène)bis(oxy)] bis-;
acide (±)-6-(carboxyéthylthio-7-éicosynoïque;
acide (±)-6-(carboxypropylthio)-7-éicosynoïque;
acide (+) et (-)-6-(carboxypentylthio)-7-éicosynoïque;
acide (±)-6-(carboxypentyloxy)-7-éicosynoïque;
acide (±)-6-[2-(N-trifluoroacétamido)-éthylthio]-7-éicosynoïque;

sel dipotassique d'acide (±)-6-(2-amino-3-hydroxy-3-oxo-propylthio)-7-éicosynoïque;
acide (±)-6-(3-hydroxy-3-oxo-2-trifluoroacétylaminopropylthio)-7-éicosynoïque;
acide 6-hydroxy-6-(1-tétradécynyl)-1,11-undécanedioïque;
acide 6-(1-tétradécynyl)-1,11-undécanedioïque;
acides 6-(1-tétradécynyl)-1,11-undec-5(E)- et (Z)ènedioïques;
acide (±)-pentanoïque, 4,4'-[(2-pentadécynylidène)bis(thio)] bis-;
acide 6-hydroxy-6-(tétradécyl)-1,11-undécanedioïque;
acide 2-butynoïque, 4,4'-[(2-pentadécynylidène)bis(oxy)]-bis-;
méthanesulfonamide, N,N'-[2-pentadécynylidène-bis(oxy-5,1-hexanediyl)]bis-; ou
acide hexanoïque, 5-[1-(5-amino-1-méthyl-5-oxopentyloxy)-2-pentadécynyloxy].

**10.** Procédé pour la préparation d'un composé ou sel revendiqué à la revendication 1, procédé dans lequel les symboles T, U, V, W X, Y $R^1$, $R^2$ $R^3$ et $R^4$, à moins que cela ne soit indiqué autrement, sont tels que définis à la revendication 1, à condition que les groupes amino, hydroxy et carboxy puissent être protégés, qui comprend :

A) pour la préparation d'un composé ou sel revendiqué à la revendication 2, la réaction d'un composé de formule X ou XA avec un composé de formule XI

$T\text{-}U\text{-}V\text{-}CR^1(OR'')_2$ X

$T\text{-}U\text{-}V\text{-}COR^1$ XA

$HWXR^2$ XI

où R'' est un groupe éthérifiant ;

B) pour la préparation d'un composé d'un sel revendiqué à la revendication 2 où un symbole W est soufre, la réaction d'un composé de formule I défini à la revendication 2 où chaque W est oxygène avec un équivalent d'un composé de formule XI défini ci-dessus où W est soufre avec déplacement du substituant $W\text{-}X\text{-}R^2$ ou $W\text{-}X\text{-}R^3$ ;

C) pour la préparation d'un composé ou sel revendiqué à la revendication 4 où W est oxygène, la réaction d'un composé de formule XII,

$T\text{-}U\text{-}V\text{-}CH(OH)YR^3$ XII,

avec une base dans un milieu solvant anhydre puis avec un composé de la formule $Hal\text{-}X\text{-}R^2$, où Hal est un atome d'halogène ;

D) pour la préparation d'un composé où sel revendiqué à la revendication 4, la réaction d'un ester d'hydrogénosulfonate d'un composé de formule XII défini ci-dessus, ou son dérivé bromo ou phosphore activé correspondant,avec un composé de formule XIV ou XV

$HS\text{-}X\text{-}R^2$ XIV

$HO\text{-}X\text{-}R^2$ XV ;

E) pour la préparation d'un composé ou sel revendiqué à la revendication 6, la réaction d'un anion dérivé d'un composé ayant la formule XVII

T-U-V-H XVII

avec un composé de formule XIX

$R^2\text{-}Y\text{-}CO\text{-}Y\text{-}R^3$ XIX ,

pour produire un composé de formule III défini à la revendication 6 où $R^4$ est un groupe hydroxyle ;

ensuite, on peut faire réagir le composé résultant de la formule III si on le souhaite, avec du trifluorure de diméthylaminosoufre pour produire le composé correspondant où $R^4$ est fluor,

ou bien il peut être déshydraté pour produire un composé où $R^4$ et un groupe Y sont combinés

pour former une double liaison ;

ensuite ce composé à double liaison peut être réduit pour produire un composé de la formule III où $R^4$ est hydrogène ou bien on peut faire réagir avec du méthylène carbène pour produire un composé correspondant ayant la formule III où $R^4$ est combiné avec un groupe Y et avec l'atome de carbone mutuellement lié pour former un noyau de cyclopropane ;

ensuite le composé résultant de la formule A où W est soufre peut,si on le souhaite être oxydé en sulfoxyde ou sulfane correspondant ;

et/ou un composé où $R^2$ ou $R^3$ est $CH_2OH$ peut être oxydé en un composé où $R^2$ au $R^3$ est $CO_2H$ ou peut être acylé en un composé où $R^2$ ou $R^3$ est $CH_2OCOCH_3$ ; et/ou les groupes protecteurs sont enlevés ;

et le composé résultant de formule A est isolé tel quel ou sous la forme de son sel acceptable en pharmacie.

**11.** Composition pharmaceutique qui comprend un

composé ayant la formule de structure A

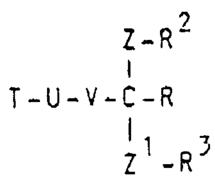

A

où T est un alkyle à chaîne droite ou ramifiée ayant 7-15 atomes de carbone qui peut facultativement contenir 1-3 doubles ou triples liaisons non cumulatives ;

U est $-CH_2CH_2-$, $-CH=CH-$ ou $-C{\equiv}C-$ ;

V est alkylène à chaîne droite ou ramifiée ayant 1 à 4 atomes de carbone ou bien est une liaison directe ;

Z et $Z^1$ sont tous deux W-X ou tous deux Y ou bien l'un est W-X et l'autre est Y, où W est O ou S-$(O)_m$ [où m est 0, 1 ou 2] ;

X est un groupe hydrocarbure divalent à chaîne droite ou ramifiée ayant 2 à 12 atomes de carbone qui peut facultativement contenir 1 à 3 doubles ou triples liaisons non cumulatives et qui peut facultativement être substitué par le groupe $-NHR^a$ [où $R^a$ est hydrogène, alkyle ayant 1 à 6 atomes de carbone, $COCF_3$, $CO(CH_2)_2CH(NH_2)CO_2H$ ou $SO_2R^b$ (où $R^b$ est alkyle ayant 1 à 6 atomes de carbone ou $CF_3$)] ;

$R^2$ et $R^3$ peuvent être identiques ou différents et sont hydrogène, $CH_2OR^c$ [où $R^c$ est hydrogène, acyle carboxylique ayant 1 à 6 atomes de carbone, tétrahydro-2H-pyran-2-yle ou $COCH_2CH_2CO_2H$], CHO, 2-tétrazolyle, $COR^d$ [où $R^d$ est hydroxy, alcoxy ayant 1 à 6 atomes de carbone. $OCH_2OC(O)C$-$(CH_3)_3$, $NHR^e$ (où $R^e$ est hydrogène, alkyle ayant 1 à 6 atomes de carbone ou $CH_2CO_2H$)] ou $SO_3H$, à condition qu'au moins l'un de $R^2$ et $R^3$ soit 2-tétrazolyle ou carboxyle ;

Y est alkylène à chaîne droite ou ramifiée ayant 1 à 12 atomes de carbone qui peut facultativement contenir 1 à 3 doubles ou triples liaisons non cumulatives ; à condition que lorsqu'un seul groupe Y est présent, il puisse facultativement être substitué par le groupe $OR^c$,où $R^c$ est tel que défini ci-dessus ; et

R est hydrogène ou, lorsque Z et $Z^1$ sont tous deux W-X, R peut également être alkyle à chaîne droite ou ramifiée ayant 1 à 6 atomes de carbone ou bien quand Z et $Z^1$ sont tous deux Y, R peut également être hydroxyle ou fluor ou bien peut être combine avec Z et avec l'atome de carbone mutuellement lié pour former une double liaison C-Z ou bien un noyau de cyclopropane ;

ou un sel acceptable en pharmacie, en association avec un support acceptable en pharmacie, à condition que lorsque R est hydrogène, U est $-C{\equiv}C-$, V est une liaison directe, $-Z,R^2$ est $-S-(CH_2)_{2-3}COOH$ et $-Z^1-R^3$ est $-S-(CH_2)_{2-3}COOH$, T soit autre que alkyle $C_{8-13}$.

**12.** Composition selon la revendication 11 sous la forme de doses unitaires.

**13.** Composé ayant la formule de structure A

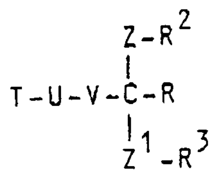

A

où T est alkyle à chaîne droite ou ramifiée ayant 7-15 atomes de carbone, qui peut facultativement contenir 1-3 doubles ou triples liaisons non cumulatives ;

U est $-CH_2CH_2-$, $-CH=CH-$ ou $-C \equiv C-$ ;

V est alkylène à chaîne droite ou ramifiée ayant 1 à 4 atomes de carbone ou bien est une liaison directe ;

Z et $Z^1$ sont tous deux W-X ou tous deux Y ou bien l'un est W-X et l'autre est Y, où W est O ou $S(O)_m$ [où m est 0, 1 ou 2] ;

X est un groupe hydrocarbure divalent à chaîne droite ou ramifiée ayant 2 à 12 atomes de carbone qui peut facultativement contenir 1 à 3 doubles ou triples liaisons non cumulatives et qui peut facultativement être substitué par le groupe $-NHR^a$ [où $R^a$ est hydrogène, alkyle ayant 1 à 6 atomes de carbone, $COCF_3$, $CO(CH_2)_2CH(NH_2)CO_2H$, ou $SO_2R^b$ (où $R^b$ est alkyle ayant 1 à 6 atomes de carbone ou $CF_3$)] ;

$R^2$ et $R^3$ peuvent être identiques ou différents et sont hydrogène, $CH_2OR^c$ [où $R^c$ est hydrogène, acyle carboxylique ayant 1 à 6 atomes de carbone, tétrahydro-2H-pyran-2-yle ou $COCH_2CH_2CO_2H$], CHO, 2-tétrazolyle, $COR^d$ [où $R^d$ est hydroxy, alcoxy ayant 1 à 6 atomes de carbone, $OCH_2OC(O)C(CH_3)_3$ , $NHR^e$ (où $R^e$ est hydrogène, alkyle ayant 1 à 6 atomes de carbone ou $CH_2CO_2H$)] ou $SO_3H$, à condition qu'au moins l'un de $R^2$ et $R^3$ soit 2-tétrazolyle ou carboxyle ;

Y est alkylène à chaîne droite ou ramifiée ayant 1 à 12 atomes de carbone, qui peut facultativement contenir 1 à 3 doubles ou triples liaisons non cumulatives ; à condition que, lorsque seul un groupe Y est présent, il puisse facultativement être substitué par le groupe $OR^c$ , où $R^c$ est tel que défini ci-dessus ; et

R est hydrogène, ou, quand Z et $Z^1$ sont tous deux W-X, R peut également être alkyle à chaîne droite ou ramifiée ayant 1 à 6 atomes de carbone ou, lorsque Z et $Z^1$ sont tous deux Y, R peut également être hydroxyle ou fluor ou bien peut être combiné avec Z et avec l'atome de carbone mutuellement lié pour former une double liaison C-Z ou un noyau de cyclopropane ;

ou son sel acceptable en pharmacie pour une utilisation dans le traitement des réactions allergiques ou du psoriasis ou d'une inflammation chez un mammifère ou pour réduire la gravité de l'infarctus du myocarde résultant d'une crise cardiaque chez un mammifère, à condition que lorsque ledit usage comprend le traitement des réactions allergiques, quand R est hydrogène, U est $-C \equiv C-$, V est une liaison directe, $-Z-R^2$ est $-S-(CH_2)_{2-3}COOH$ et $Z^1-R^3$ est $-S-(CH_2)_{2-3}COOH$, T soit autre autre que alkyle $C_{8-13}$.

**14.** Composé choisi parmi ceux qui suivent :
1-butanol, 4,4'-[(2-nonynylidène)bis(oxy)]bis-,dibenzoate;
1-butanol, 4,4'-[(2-nonynylidène)bis(oxy)]bis-;
acide butanoïque, 4,4'-[(2-nonynylidène)bis(oxy)]bis;
L-cystéine, S[R ou S]-[1-(4-méthoxy-4-oxobutoxy)-2-nonynyl]-N-(trifluoroacétyl)-, ester méthylique - (isomère le moins polaire); et
L-cystéine S[R ou S]-[1-(4-méthoxy-4-oxobutoxy)-2-nonynyl]-N-(trifluoroacétyl)-, ester méthylique - (isomère le plus polaire).

## Patentansprüche

**1.** Verbindung mit der Strukturformel A

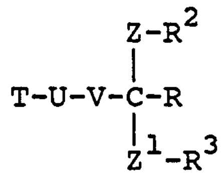

A

worin T ein gerad- oder verzweigt-kettiges Alkyl mit 7-15 Kohlenstoff-Atomen ist, die je nach Wahl 1-3 nicht-kumulierte Doppel- oder Dreifachbindungen enthalten können;

U $-CH_2CH_2-$, $-CH=CH-$ oder $-C\equiv C-$ ist;

V ein gerad- oder verzweigt-kettiges Alkylen mit 1 bis 4 Kohlenstoff-Atomen oder eine direkte Bindung ist;

Z und $Z^1$ beide W-X oder beide y sind, oder eines W-X und das andere Y ist, wobei W für O oder S-$(O)_m$ steht (worin m 0, 1 oder 2 ist);

X eine gerad- oder verzweigt-kettige bivalente Kohlenwasserstoff-Gruppe mit 2 bis 12 Kohlenstoff-Atomen ist, die je nach Wahl 1 bis 3 nicht-kumulierte Doppel- oder Dreifachbindungen enthalten kann, und die wahlweise mit der Gruppe $-NHR^a$ substituiert sein kann [worin $R^a$ Wasserstoff, Alkyl mit 1 bis 6 Kohlenstoff-Atomen, $COCF_3$, $CO(CH_2)_2CH(NH_2)CO_2H$ oder $SO_2R^b$ ist (worin $R^b$ Alkyl mit 1 bis 6 Kohlenstoff-Atomen oder $CF_3$ ist)];

$R^2$ und $R^3$ gleich oder verschieden sein können und Wasserstoff sind, $CH_2OR^c$ [worin $R^c$ Wasserstoff, Carbonsäureacyl mit 1 bis 6 Kohlenstoff-Atomen, Tetrahydro-2H-pyran-2-yl oder $COCH_2CH_2CO_2H$ ist], CHO, 2-Tetrazolyl, $COR^d$ [worin $R^d$ Hydroxy, Alkoxy mit 1 bis 6 Kohlenstoff-Atomen, $OCH_2OC(O)C(CH_3)_3$, $NHR^e$ ist (worin $R^e$ Wasserstoff, Alkyl mit 1 bis 6 Kohlenstoff-Atomen oder $CH_2CO_2H$ ist)] oder $SO_3H$, mit der Maßgabe, daß wenigstens eines der $R^2$ und $R^3$ 2-Tetrazolyl oder Carboxyl ist;

Y ein gerad- oder verzweigt-kettiges Alkylen mit 1 bis 12 Kohlenstoff-Atomen ist, die je nach Wahl 1 bis 3 nichtkumulierte Doppel- oder Dreifachbindungen enthalten können; mit der Maßgabe, daß, wenn nur eine Gruppe Y vorhanden ist, diese wahlweise mit der Gruppe $OR^c$ substituiert sein kann, wobei $R^c$ wie oben definiert ist;

und
R Wasserstoff ist, oder, wenn Z und $Z^1$ beide W-X sind, R auch gerad- oder verzweigt-kettiges Alkyl mit 1 bis 6 Kohlenstoff-Atomen sein kann, oder, wenn Z und $Z^1$ beide Y sind, R auch Hydroxyl oder Fluor sein kann oder mit Z und dem gemeinsam gebundenen Kohlenstoff-Atom kombiniert sein kann, unter Bildung einer C-Z-Doppelbindung oder eines Cyclopropan-Rings;

und deren pharmazeutisch annehmbare Salze;

mit den folgenden Maßgaben:
(A) wenn R Wasserstoff ist, ist U $-C\equiv C-$, V eine direkte Bindung, $-Z-R^2$ ist $-S-(CH_2)_{2-3}COOH$ und $-Z^1-R^3$ ist $-S-(CH_2)_{2-3}COOH$, und T ist verschieden von $C_{8-13}$-Alkyl;
(B) die Verbindung der Formel A ist nicht Isodecan-1,1-bis(mercaptopropionsäure).

**2.** Verbindung nach Anspruch 1 mit der Strukturformel

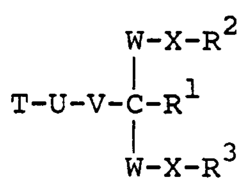

I

worin T, U, V, W, $R^2$ und $R^3$ wie in Anspruch 1 definiert sind, X ein gerad- oder verzweigt-kettiges Alkylen mit 2 bis 12 Kohlenstoff-Atomen ist, die je nach Wahl 1 bis 3 nichtkumulierte Doppel- oder Dreifachbindungen enthalten können, und die wahlweise mit der Gruppe $-NHR^a$ (worin $R^a$ wie in Anspruch 1 definiert ist) substituiert sein können; und $R^1$ Wasserstoff oder ein gerad- oder verzweigt-kettiges Alkyl mit 1-6 Kohlenstoff-Atomen ist; und deren pharmazeutisch annehmbare Salze.

**3.** Verbindung nach Anspruch 2, worin
T ein geradkettiges Alkyl mit 7-15 Kohlenstoff-Atomen ist;
U -C≡C- ist;
V eine direkte Bindung ist;
W jeweils O ist;
X jeweils ein gerad- oder verzweigt-kettiges Alkylen mit 2 bis 8 Kohlenstoff-Atomen ist;
$R^1$ Wasserstoff ist; und
$R^2$ und $R^3$ COOH sind;
und deren pharmazeutisch annehmbare Salze mit Basen.

**4.** Verbindung nach Anspruch 1 mit der Strukturformel

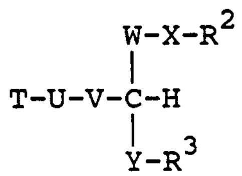

II

worin T, U, V, W, X, Y, $R^2$ und $R^3$ wie in Anspruch 1 definiert sind; und deren pharmazeutisch annehmbare Salze.

**5.** Verbindung nach Anspruch 4, worin
T ein geradkettiges Alkyl mit 7-15 Kohlenstoff-Atomen ist;
U -C≡C- ist;
V eine direkte Bindung ist;
W O oder S ist;
X und Y gerad- oder verzweigt-kettige Alkylene mit 2 bis 6 Kohlenstoff-Atomen sind; und
$R^2$ und $R^3$ COOH sind;
und deren pharmazeutisch annehmbare Salze mit Basen.

**6.** Verbindung nach Anspruch 1 mit der Strukturformel

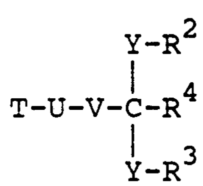

III

worin T, U, V, Y, $R^2$ und $R^3$ wie in Anspruch 1 definiert sind; und $R^4$ Wasserstoff oder Hydroxyl ist, oder mit einer Gruppe Y und mit dem gemeinsam gebundenen Kohlenstoff-Atom kombiniert werden kann unter Bildung einer C-Y-Doppelbindung oder eines Cyclopropan-Rings; und deren pharmazeutisch annehmbare Salze.

**7.** Verbindung nach Anspruch 6, worin
T ein geradkettiges Alkyl mit 7-15 Kohlenstoff-Atomen ist;
U -C≡C- ist;
V eine direkte Bindung ist;

Y jeweils ein gerad- oder verzweigt-kettiges Alkylen mit 2 bis 6 Kohlenstoff-Atomen ist;
$R^2$ und $R^3$ COOH sind; und
$R^4$ Wasserstoff oder Hydroxyl ist, oder mit einer Gruppe Y und mit dem gemeinsam gebundenen Kohlenstoff-Atom kombiniert werden kann unter Bildung einer Doppelbindung;
und deren pharmazeutisch annehmbare Salze mit Basen.

**8.** Verbindung nach irgendeinem der Ansprüche 2 bis 7, worin die Substituenten T-U-V- zusammengenommen die 1-Tetradecinyl-Gruppe bilden, d.h., $n\text{-}C_{12}H_{25}C{\equiv}C\text{-}$, und/oder worin $R^2$ und $R^3$ gleich oder verschieden sein können und $COR^d$ sind, worin $R^d$ wie in Anspruch 1 definiert ist.

**9.** Verbindung nach Anspruch 1 mit folgender Bezeichnung:
Butansäure, 4,4'-[(2-Pentadecinyliden)bis(oxy)]bis-;
Hexansäure, 6,6'-[(2-Pentadecinyliden)bis(oxy)]bis-;
(±)-Pentansäure, 4,4'-[(2-Pentadecinyliden)bis(oxy)]-bis-;
(±)-Heptansäure, 6,6'-[(2-Pentadecinyliden)bis(oxy)]-bis-;
(±)-6-(4-Hydroxybutoxy)-5-oxaeicos-7-insäure;
Hexansäure, 6,6'-[(2-Pentadecinyliden)bis(thio)]bis-;
Butansäure, 4,4'-[(Tridecyliden)bis(oxy)]bis-;
Butansäure, 4,4'-[(Pentadecyliden)bis(oxy)]bis-;
(±)-Hexansäure, 4,4'-[(2-Pentadecinyliden)bis(oxy)]bis-;
(±)-6-(Carboxyethylthio)-7-eicosinsäure;
(±)-6-(Carboxypropylthio)-7-eicosinsäure;
(+) und (-)-6-(Carboxypentylthio)-7-eicosinsäure;
(±)-6-(Carboxypentyloxy)-7-eicosinsäure;
(±)-6-[2-(N-Trifluoracetamido)-ethylthio]-7-eicosinsäure;
(±)-6-(2-Amino-3-hydroxy-3-oxopropylthio)-7-eicosinsäure, Dikalium-Salz;
(±)-6-(3-Hydroxy-3-oxo-2-trifluoracetylamino-propylthio)-7-eicosinsäure;
6-Hydroxy-6-(1-tetradecinyl)-1,11-undecandisäure;
6-(1-Tetradecinyl)-1,11-undecandisäure;
6-(1-Tetradecinyl)-1,11-undec-5(E)- und -(Z)-endisäure;
(±)-Pentansäure, 4,4'-[(2-Pentadecinyliden)bis(thio)]bis-;
6-Hydroxy-6-(tetradecyl)-1,11-undecandisäure;
2-Butinsäure, 4,4'-[(2-Pentadecinyliden)bis(oxy)]bis-;
Methansulfonamid, N,N'-[2-Pentadecinyliden-bis(oxy-5,1-hexandiyl)]bis-; oder
Hexansäure, 5-[1-(5-Amino-1-methyl-5-oxopentyloxy)-2-pentadecinyloxy].

**10.** Verfahren zur Herstellung einer Verbindung oder eines Salzes nach Anspruch 1, bei welchem die Symbole T, U, V, W, X, Y, $R^1$, $R^2$, $R^3$ und $R^4$ - wenn nicht anders vermerkt - wie in Anspruch 1 definiert sind, mit der Maßgabe, daß Amino-, Hydroxy- und Carboxy-Gruppen gschützt werden können, umfassend

A) zur Herstellung einer Verbindung oder eines Salzes nach Anspruch 2, die Umsetzung einer Verbindung der Formel X oder XA mit einer Verbindung der Formel XI

$T\text{-}U\text{-}V\text{-}CR^1(OR'')_2$ X

$T\text{-}U\text{-}V\text{-}COR^1$ XA

$HWXR^2$ XI

worin R'' eine verethernde Gruppe ist;

B) zur Herstellung einer Verbindung oder eines Salzes nach Anspruch 2, worin ein Symbol W Schwefel ist, die Umsetzung einer Verbindung der Formel I wie in Anspruch 2 definiert, wo W jeweils Sauerstoff ist, mit einem Äquivalent einer Verbindung der Formel XI wie oben definiert, worin W Schwefel ist, unter Ersetzung des Substituenten $W\text{-}X\text{-}R^2$ oder $W\text{-}X\text{-}R^3$;

C) zur Herstellung einer Verbindung oder eines Salzes nach Anspruch 4, worin W Sauerstoff ist, die Umsetzung einer Verbindung der Formel XII

$T\text{-}U\text{-}V\text{-}CH(OH)YR^3$ XII,

mit einer Base in einem wasserfreien Lösungsmittelmedium, und dann mit einer Verbindung der Formel Hal-X-$R^2$, worin Hal ein Halogen-Atom ist;

D) zur Herstellung einer Verbindung oder eines Salzes nach Anspruch 4, die Umsetzung eines Hydrogensulfonatesters einer Verbindung der Formel XII wie oben definiert, oder des entsprechenden Brom- oder aktivierten Phosphor-Derivats, mit einer Verbindung der Formel XIV oder XV

HS-X-$R^2$ XIV

HO-X-$R^2$ XV;

E) zur Herstellung einer Verbindung oder eines Salzes nach Anspruch 6, die Umsetzung eines Anions, abgeleitet aus einer Verbindung der Formel XVII

T-U-V-H XVII

mit einer Verbindung der Formel XIX

$R^2$-Y-CO-Y-$R^3$ XIX,

unter Bildung einer Verbindung der Formel III wie in Anspruch 6 definiert, worin $R^4$ eine Hydroxyl-Gruppe ist;

wonach die gebildete Verbindung der Formel III, wenn gewünscht, mit Diethylaminoschwefeltrifluorid umgesetzt werden kann, unter Bildung der entsprechenden Verbindung, worin $R^4$ Fluor ist,
oder dehydratisiert werden kann, unter Bildung einer Verbindung, worin $R^4$ und eine Gruppe Y zusammen eine Doppelbindung bilden;
wonach diese Verbindung mit Doppelbindung reduziert werden kann, unter Bildung einer Verbindung der Formel III, worin $R^4$ Wasserstoff ist, oder umgesetzt werden kann mit Methylencarben, unter Bildung einer entsprechenden Verbindung der Formel III, worin $R^4$ mit einer Gruppe Y und dem gemeinsam gebundenen Kohlenstoff-Atom kombiniert ist, unter Bildung eines Cyclopropan-Ringes;
wonach die gebildete Verbindung der Formel A, worin W Schwefel ist, wenn gewünscht zum entsprechenden Sulfoxid oder Sulfon oxidiert werden kann;
und/oder eine Verbindung, worin $R^2$ oder $R^3$ $CH_2OH$ ist, oxidiert werden kann zu einer Verbindung, worin $R^2$ oder $R^3$ $CO_2OH$ ist, oder acyliert werden kann zu einer Verbindung, worin $R^2$ oder $R^3$ $CH_2OCOCH_3$ ist;
und/oder alle Schutzgruppen entfernt werden;
und die gebildete Verbindung der Formel A als solche isoliert wird oder als deren pharmazeutisch annehmbares Salz.

**11.** Pharmazeutische Zusammensetzung, die eine Verbindung der Strukturformel A umfaßt

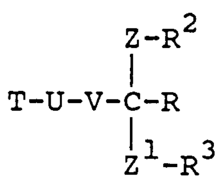

A

worin T ein gerad- oder verzweigt-kettiges Alkyl mit 7-15 Kohlenstoff-Atomen ist, die je nach Wahl 1-3 nichtkumulierte Doppel- oder Dreifachbindungen enthalten können;

U $-CH_2CH_2-$, -CH=CH- oder -C≡C- ist;

V ein gerad- oder verzweigt-kettiges Alkylen mit 1 bis 4 Kohlenstoff-Atomen oder eine direkte Bindung ist;

Z und $Z^1$ beide W-X oder beide Y sind, oder eines W-X und das andere Y ist, wobei W für O oder S-$(O)_m$ steht (worin m 0, 1 oder 2 ist);

X eine gerad- oder verzweigt-kettige bivalente Kohlenwasserstoff-Gruppe mit 2 bis 12 Kohlenstoff-Atomen ist, die je nach Wahl 1 bis 3 nicht-kumulierte Doppel- oder Dreifachbindungen enthalten kann, und die wahlweise mit der Gruppe $-NHR^a$ substituiert sein kann [worin $R^a$ Wasserstoff, Alkyl mit 1 bis 6 Kohlenstoff-Atomen, $COCF_3$, $CO(CH_2)_2CH(NH_2)CO_2H$ oder $SO_2R^b$ ist (worin $R^b$ Alkyl mit 1 bis 6 Kohlenstoff-Atomen oder $CF_3$ ist)];

$R^2$ und $R^3$ gleich oder verschieden sein können und Wasserstoff sind, $CH_2OR^c$ [worin $R^c$ Wasserstoff, Carbonsäureacyl mit 1 bis 6 Kohlenstoff-Atomen, Tetrahydro-2H-pyran-2-yl oder $COCH_2CH_2CO_2H$ ist], CHO, 2-Tetrazolyl, $COR^d$ [worin $R^d$ Hydroxy, Alkoxy mit 1 bis 6 Kohlenstoff-Atomen, $OCH_2OC(O)C(CH_3)_3$, $NHR^e$ ist (worin $R^e$ Wasserstoff, Alkyl mit 1 bis 6 Kohlenstoff-Atomen oder $CH_2CO_2H$ ist)] oder $S_3O_3H$, mit der Maßgabe, daß wenigstens eines der $R^2$ und $R^3$ 2-Tetrazolyl oder Carboxyl ist;

Y ein gerad- oder verzweigt-kettiges Alkylen mit 1 bis 12 Kohlenstoff-Atomen ist, die je nach Wahl 1 bis 3 nicht-kumulierte Doppel- oder Dreifachbindungen enthalten können; mit der Maßgabe, daß, wenn nur eine Gruppe Y vorhanden ist, diese wahlweise mit der Gruppe $OR^c$ substituiert werden kann, wobei $R^c$ wie oben definiert ist;

und

R Wasserstoff ist, oder, wenn Z und $Z^1$ beide W-X sind, R auch ein gerad- oder verzweigt-kettiges Alkyl mit 1 bis 6 Kohlenstoff-Atomen sein kann, oder, wenn Z und $Z^1$ beide Y sind, R auch Hydroxyl oder Fluor sein kann, oder mit Z und dem gemeinsam gebundenen Kohlenstoff-Atom kombiniert sein kann, unter Bildung einer C-Z-Doppelbindung oder eines Cyclopropan-Rings;

oder deren pharmazeutisch annehmbares Salz, in Verbindung mit einem pharmazeutisch annehmbaren Träger, mit der Maßgabe, daß, wenn R Wasserstoff ist, U -C≡C- ist, V eine direkte Bindung, $-Z-R^2$ für $-S-(CH_2)_{2-3}COOH$ und $-Z^1-R^3$ für $-S-(CH_2)_{2-3}COOH$ steht, und T verschieden von $C_{8-13}$-Alkyl ist.

**12.** Zusammensetzung nach Anspruch 11 in Form von Dosiereinheiten.

**13.** Verbindung der Strukturformel A

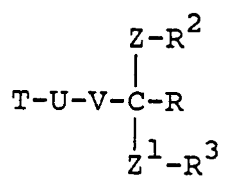

A

worin T ein gerad- oder verzweigt-kettiges Alkyl mit 7-15 Kohlenstoff-Atomen ist, die je nach Wahl 1-3 nichtkumulierte Doppel- oder Dreifachbindungen enthalten können;

U $-CH_2CH_2-$, -CH=CH- oder -C≡C- ist;

V ein gerad- oder verzweigt-kettiges Alkylen mit 1 bis 4 Kohlenstoff-Atomen oder eine direkte Bindung ist;

Z und $Z^1$ beide W-X oder beide Y sind, oder eines W-X und das andere Y ist, wobei W für O oder S-$(O)_m$ steht (worin m 0, 1 oder 2 ist);

X eine gerad- oder verzweigt-kettige bivalente Kohlenwasserstoff-Gruppe mit 2 bis 12 Kohlenstoff-Atomen ist, die je nach Wahl 1 bis 3 nicht-kumulierte Doppel- oder Dreifachbindungen enthalten kann, und die wahlweise mit der Gruppe $-NHR^a$ substituiert sein kann [worin $R^a$ Wasserstoff, Alkyl mit 1 bis 6 Kohlenstoff-Atomen, $COCF_3$, $CO(CH_2)_2CH(NH_2)CO_2H$ oder $SO_2R^b$ ist (worin $R^b$ Alkyl mit 1 bis 6 Kohlenstoff-Atomen oder $CF_3$ ist)];

$R^2$ und $R^3$ gleich oder verschieden sein können und Wasserstoff sind, $CH_2OR^c$ [worin $R^c$ Wasserstoff, Carbonsäureacyl mit 1 bis 6 Kohlenstoff-Atomen, Tetrahydro-2H-pyran-2-yl oder $COCH_2CH_2CO_2H$ ist], CHO, 2-Tetrazolyl, $COR^d$ [worin $R^d$ Hydroxy, Alkoxy mit 1 bis 6 Kohlenstoff-Atomen, $OCH_2OC(O)C(CH_3)_3$, $NHR^e$ ist (worin $R^e$ Wasserstoff, Alkyl mit 1 bis 6 Kohlenstoff-Atomen oder $CH_2CO_2H$ ist)] oder $SO_3H$, mit der Maßgabe, daß wenigstens eines der $R^2$ und $R^3$ 2-Tetrazolyl oder Carboxyl ist;

Y ein gerad- oder verzweigt-kettiges Alkylen mit 1 bis 12 Kohlenstoff-Atomen ist, die je nach Wahl 1 bis 3 nicht-kumulierte Doppel- oder Dreifachbindungen enthalten können; mit der Maßgabe, daß, wenn nur eine Gruppe Y vorhanden ist, diese wahlweise mit der Gruppe $OR^c$ substituiert werden kann, wobei $R^c$ wie oben definiert ist;

und

R Wasserstoff ist, oder, wenn Z und $Z^1$ beide W-X sind, R auch ein gerad- oder verzweigt-kettiges Alkyl mit 1 bis 6 Kohlenstoff-Atomen sein kann, oder, wenn Z und $Z^1$ beide Y sind, R auch Hydroxyl oder Fluor sein kann, oder mit Z und dem gemeinsam gebundenen Kohlenstoff-Atom kombiniert sein kann, unter Bildung einer C-Z-Doppelbindung oder eines Cyclopropan-Rings;

oder deren pharmazeutisch annehmbares Salz, zur Verwendung für die Behandlung von allergischen Reaktionen oder Schuppenflechte oder Entzündungen bei Säugetieren, oder zur Minderung der Schwere von Myokardinfarkten, die aus Herzattacken bei Säugetieren resultieren, mit der Maßgabe, daß, wenn die Verwendung die Behandlung von allergischen Reaktionen umfaßt, R Wasserstoff ist, U -C≡C- ist, V eine direkte Bindung, $-Z-R^2$ für $-S-(CH_2)_{2-3}COOH$und $-Z^1-R^3$ für $-S-(CH_2)_{2-3}COOH$ steht, und T verschieden von $C_{8-13}$-Alkyl ist.

**14.** Verbindung, ausgewählt aus den folgenden:
1-Butanol, 4,4'-[(2-Noninyliden)bis(oxy)]bis-, Dibenzoat;
1-Butanol, 4,4'-[(2-Noninyliden)bis(oxy)]bis-, Butansäure, 4,4'-[(2-Noninyliden)bis(oxy)]bis-;
L-Cystein, S[R oder S]-[1-(4-Methoxy-4-oxobutoxy)-2-noninyl]-N-(trifluoracetyl)-, Methylester (weniger polares Isomer); und
L-Cystein, S[R oder S]-[1-(4-Methoxy-4-oxobutoxy)-2-noninyl]-N-(trifluoracetyl)-, Methylester (stärker polares Isomer).